# EUROPEAN PATENT SPECIFICATION

(11) **EP 1 135 532 B1**
(45) Date of publication and mention of the grant of the patent: **16.03.2011**
(21) Application number: 99966135.8
(22) Date of filing: 10.12.1999
(51) Int. Cl.: C12Q 1/68, C07K 14/435

(54) **FLUORESCENT PROTEINS FROM NON-BIOLUMINESCENT SPECIES OF CLASS ANTHOZOA, GENES ENCODING SUCH PROTEINS AND USES THEREOF**
FLUORESZIERENDE PROTEINE AUS NICHT-BIOLUMINESZENTEN ARTEN DER KLASSE ANTHOZOA, GENE DIE DIESE PROTEINE KODIEREN UND DEREN VERWENDUNGEN
PROTEINES FLUORESCENTES APPARTENANT A DES ESPECES NON BIOLUMINESCENTES DE LA CLASSE DES ANTHOZOAIRES, GENES CODANT POUR CES PROTEINES ET UTILISATIONS

(30) Priority: 11.12.1998 US 210330
(43) Date of publication of application: 26.09.2001
(73) Proprietor: Clontech Laboratories Inc., Palo Alto, CA 94303 (US)
(72) Inventor: LUKYANOY, Sergey Anatolievich, Moscow (RU); FRADKOV, Arcady Fedorovich, Moscow, 113570 (RU); LABAS, Yulii Aleksandrovich, Moscow, 117465 (RU); MATZ, Mikhail Vladimirovich, Moscow, 117465 (RU)
(74) Representative: Brasnett, Adrian Hugh
(86) International application number: PCT/US1999/029405
(87) International publication number: WO 2000/034526

(56) References cited:
- WO-A-00/34318
- WO-A-00/34319
- WO-A-00/34320
- WO-A-00/34321
- WO-A-00/34322
- WO-A-00/34323
- WO-A-00/34324
- WO-A-00/34325
- WO-A-00/34326
- DE-A1- 19 718 640
- US-A1- 2005 032 085
- ANDERLUH G ET AL: "CLONING, SEQUENCING, AND EXPRESSION OF EQUINATOXIN II" BIOCHEMICAL AND BIOPHYSICAL RESEARCH COMMUNICATIONS, ACADEMIC PRESS INC. ORLANDO, FL, US, vol. 220, 1996, pages 437-442, XP002923209 ISSN: 0006-291X
- MACEK P ET AL: "INTRINSIC TRYPTOPHAN FLUORESCENCE OF EQUINATOXIN II, A PORE-FORMING POLYPEPTIDE FROM THE SEA ANEMONE ACTINIA EQUINA L, MONITORS ITS INTERACTION WITH LIPID MEMBRANES" EUROPEAN JOURNAL OF BIOCHEMISTRY, BERLIN, DE, vol. 234, 1995, pages 329-335, XP002923207 ISSN: 0014-2956
- WARD W W ET AL: "AN ENERGY TRANSFER PROTEIN IN COELENTERATE BIOLUMINESCENCE CHARACTERIZATION OF THE RENILLA GREEN-FLUORESCENT PROTEIN" JOURNAL OF BIOLOGICAL CHEMISTRY. (MICROFILMS), AMERICAN SOCIETY OF BIOLOGICAL CHEMISTS, BALTIMORE, MD, US, vol. 254, no. 3, 10 February 1979 (1979-02-10), pages 781-788, XP002030243
- TSIEN R: "Posy dawn for fluoresent proteins" NATURE BIOTECHNOLOGY, vol. 17, October 1999 (1999-10), pages 956-957, XP002294928
- MATZ M.V. ET AL: 'Fluorescent Proteins from Nonbioluminescent Anthozoa Species' NATURE BIOTECHNOLOGY vol. 17, October 1999, pages 969 - 973, XP002922104
- DATABASE NUCLEOTIDE [Online] 21 October 1999 'Discosoma sp. flourescent protein FP583 mRNA, complete cds' Retrieved from NCBI Database accession no. AF168419
- DATABASE NUCLEOTIDE [Online] 21 October 1999 'Discosoma striata fluorescent protein FP483 mRNA, complete cds' Retrieved from NCBI Database accession no. AF168420
- DATABASE NUCLEOTIDE [Online] 21 October 1999 'Anemonia majano fluorescent protein FP486 mRNA, complete cds' Retrieved from NCBI Database accession no. AF168421
- WIEDENMANN J. ET AL: 'Cracks in the beta-can: Fluorescent proteins from Anemonia sulcata (Anthozoa, Actinaria)' PNAS vol. 97, no. 26, 19 December 2000, pages 14091 - 14096
- WIEDENMANN J.: 'Zur Biologie der Ökotypen von Annemonia sulcata (Pennant)' DIPLOMARBEIT 1996, ULM,
- PRASHER ET AL: 'Primary structure of the Aequorea victoria green fluorescent protein' GENE vol. 111, 1992, pages 229 - 233
- DATABASE NUCLEOTIDE [Online] 19 November 2000 'Anemonia sulcata green fluorescent protein as FP499 mRNA, complete cds' Retrieved from NCBI Database accession no. AF322221.1
- DATABASE NUCLEOTIDE [Online] 28 February 2002 'Anemonia sulcata nonfluorescent red protein asCP562 mRNA, complete cds' Retrieved from NCBI Database accession no. AF322222.2
- MAZEL: 'Spectral measurements of fluorescence emissions' MARINE ECOLOGY PROGRESS SERIES vol. 120, 20 April 1995, pages 185 - 191
- WARD ET AL: 'Spectophotometric identity of the energy transfer chromophores in renilla and aequorea green-fluorescent proteins' PHOTOCHEMISTRY AND PHOTOBIOLOGY vol. 31, no. 6, 1980, pages 611 - 616 ISSN: 0031-8655
- CUBITT A.B. ET AL: 'Understanding, improving and using green fluorescent proteins' TRENDS IN BIOCHEMICAL SCIENCES, ELSEVIER, HAYWARDS, GB LNKD- DOI:10.1016/S0968-0004(00)89099-4 vol. 20, no. 11, 01 November 1995, pages 448 - 455, XP004222335 ISSN: 0968-0004
- BOKMAN S.H. ET AL: 'Renaturation of Aequorea green-fluorescent protein' BIOCHEMICAL AND BIOPHYSICAL RESEARCH COMMUNICATIONS, ACADEMIC PRESS INC. ORLANDO, FL, US LNKD- DOI:10.1016/0006-291X(81)91599-0 vol. 101, no. 4, 31 August 1981, pages 1372 - 1380, XP024776176 ISSN: 0006-291X
- WILSON T. ET AL: 'Bioluminescence' ANNUAL REVIEW OF CELL AND DEVELOPMENTAL BIOLOGY 1998 LNKD- PUBMED:9891783 vol. 14, 1998, pages 197 - 230 ISSN: 1081-0706
- BAIRD G.S. ET AL: 'Biochemistry, mutagenesis, and oligomerization of DsRed, a red fluorescent protein from coral.' PROCEEDINGS OF THE NATIONAL ACADEMY OF SCIENCES OF THE UNITED STATES OF AMERICA 24 OCT 2000 LNKD- PUBMED:11050229 vol. 97, no. 22, 24 October 2000, pages 11984 - 11989 ISSN: 0027-8424
- Dr. David Gruber: "Function of fluorescent proteins in corals" 14 June 2009 (2009-06-14), Retrieved from the Internet: URL:http://reefresearch.org/ccmi_website/e dufield/edufield_05_05.htm
- BEVIS BROOKE J. ET AL: 'Rapidly maturing variants of the Discosoma red fluorescent protein (DsRed).' NATURE BIOTECHNOLOGY JAN 2002 LNKD- PUBMED:11753367 vol. 20, no. 1, January 2002, pages 83 - 87 ISSN: 1087-0156
- CAMPBELL R.E. ET AL: 'A monomeric red fluorescent protein' PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.082243699 vol. 99, no. 12, 11 June 2002, pages 7877 - 7882, XP002967837 ISSN: 0027-8424
- SHANER NATHAN C. ET AL: 'Improved monomeric red, orange and yellow fluorescent proteins derived from Discosoma sp. red fluorescent protein.' NATURE BIOTECHNOLOGY DEC 2004 LNKD- PUBMED:15558047 vol. 22, no. 12, December 2004, pages 1567 - 1572 ISSN: 1087-0156

### SEQUENCE LISTING

<110> Lukyanov, Sergey A.
   Labas, Yulii A.
   Matz, Mikhail V.
   Fradkov, Arcady F.
<120> Fluorescent proteins from non-bioluminescent species of Class Anthozoa, genes encoding such proteins and uses thereof
<130> D6196PCT
<141> 1999-12-10
<150> 09/210,330
   <151> 1998-12-11
<160> 63
<210> 1
   <211> 25
   <212> DNA
   <213> artificial sequence
<220>
   <221> primer_bind
   <223> primer TN3 used in cDNA synthesis and RACE
<400> 1
   cgcagtcgac cgtttttttt ttttt 25
<210> 2
   <211> 23
   <212> DNA
   <213> artificial sequence
<220>
   <221> primer_bind
   <223> primer TS used in cDNA synthesis and RACE
<400> 2
   aagcagtggt atcaacgcag agt 23
<210> 3
   <211> 6
   <212> PRT
   <213> *Aequorea victoria*
<220>
   <222> 21
   <223> amino acid sequence of a key stretch on which primer NGH is based; Xaa at position 21 represents unknown
<400> 3
   Gly Xaa Val Asn Gly His
   5
<210> 4
   <211> 20
   <212> DNA
   <213> artificial sequence
<220>
   <221> primer_bind
   <222> 12
   <223> primer NGH used for isolation of fluorescent protein; n at position 12 represents any of the four bases
<400> 4
   gayggctgcg tnaayggdca 20
<210> 5
   <211> 5
   <212> PRT
   <213> *Aequorea victoria*
<220>
   <222> 31..35
   <223> amino acid sequence of a key stretch on which primers GEGa and GEGb are based
<400> 5
   Gly Glu Gly Glu Gly
                   5
<210> 6
   <211> 20
   <212> DNA
   <213> artificial sequence
<220>
   <221> primer_bind
   <223> primer GEGa used for isolation of fluorescent protein
<400> 6
   gttacaggtg arggmgargg 20
<210> 7
   <211> 20
   <212> DNA
   <213> artificial sequence
<220>
   <221> primer_bind
   <223> primer GEGb used for isolation of fluorescent protein
<400> 7
   gttacaggtg arggkgargg 20
<210> 8
   <211> 5
   <212> PRT
   <213> *Aequorea victoria*
<220>
   <222> 31...35
   <223> amino acid sequence of a key stretch on which primers GNGa and GNGb are based
<400> 8
   Gly Glu Gly Asn Gly
                   5
<210> 9
   <211> 20
   <212> DNA
   <213> artificial sequence
<220>
   <221> primer_bind
   <223> primer GNGa used for isolation of fluorescent protein
<400> 9
   gttacaggtg arggmaaygg 20
<210> 10
   <211> 20
   <212> DNA
   <213> artificial sequence
<220>
   <221> primer_bind
   <223> primer GNGb used for isolation of fluorescent protein
<400> 10
   gttacaggtg arggkaaygg 20
<210> 11
   <211> 5
   <212> PRT
   <213> *Aequorea victoria*
<220>
   <222> 127..131
   <223> amino acid sequence of a key stretch on which primer NFP is based
<400> 11
   Gly Met Asn Phe Pro
   5
<210> 12
   <211> 5
   <212> PRT
   <213> *Aequorea victoria*
<220>
   <222> 127...131
   <223> amino acid sequence of a key stretch on which primer NFP is based
<400> 12
   Gly Val Asn Phe Pro
   5
<210> 13
   <211> 20
   <212> DNA
   <213> artificial sequence
<220>
   <221> primer_bind
   <223> primer NFP used for isolation of fluorescent protein
<400> 13
   ttccayggtr tgaayttycc 20
<210> 14
   <211> 4
   <212> PRT
   <213> *Aequorea victoria*
<220>
   <222> 134..137
   <223> amino acid sequence of a key stretch on which primers PVMa and PVMb are based
<400> 14
   Gly Pro Val Met
<210> 15
   <211> 21
   <212> DNA
   <213> artificial sequence
<220>
   <221> primer_bind
   <222> 15
   <223> primer PVMa used for isolation of fluorescent protein; n at position 15 represents any of the four bases
<400> 15
   cctgccrayg gtccngtmat g 21
<210> 16
   <211> 21
   <212> DNA
   <213> artificial sequence
<220>
   <221> primer_bind
   <222> 15
   <223> primer PVMb used for isolation of fluorescent protein; n at position 15 represents any of the four bases
<400> 16
   cctgccrayg gtccngtkat g 21
<210> 17
   <211> 47
   <212> DNA
   <213> artificial sequence
<220>
   <221> primer_bind
   <223> primer T7-TN3 used in cDNA synthesis and RACE
<400> 17
   gtaatacgac tcactatagg gccgcagtcg accgtttttt ttttttt 47
<210> 18
   <211> 45
   <212> DNA
   <213> artificial sequence
<220>
   <221> primer_bind
   <223> primer T7-TS used in cDNA synthesis and RACE
<400> 18
   gtaatacgac tcactatagg gcaagcagtg gtatcaacgc agagt 45
<210> 19
   <211> 22
   <212> DNA
   <213> artificial sequence
<220>
   <221> primer-bind
   <223> primer T7 used in cDNA synthesis and RACE
<400> 19
   gtaatacgac tcactatagg gc 22
<210> 20
   <211> 21
   <212> DNA
   <213> artificial sequence
<220>
   <221> primer-bind
   <223> gene-specific primer used for 5'-RACE for *Anemonia majano*
<400> 20
   gaaatagtca ggcatactgg t 21
<210> 21
   <211> 20
   <212> DNA
   <213> artificial sequence
<220>
   <221> primer_bind
   <223> gene-specific primer used for 5'-RACE for *Anemonia majano*
<400> 21
   gtcaggcata ctggtaggat 20
<210> 22
   <211> 21
   <212> DNA
   <213> artificial sequence
<220>
   <221> primer_bind
   <223> gene-specific primer used for 5'-RACE for *Clavularia sp.*
<400> 22
   cttgaaatag tctgctatat c 21
<210> 23
   <211> 19
   <212> DNA
   <213> artificial sequence
<220>
   <221> primer_bind
   <223> gene-specific primer used for 5'-RACE for *Clavularia sp.*
<400> 23
   tctgctatat cgtctgggt 19
<210> 24
   <211> 23
   <212> DNA
   <213> artificial sequence
<220>
   <221> primer_bind
   <223> gene-specific primer used for 5'-RACE for Zoanthus sp.
<400> 24
   gttcttgaaa tagtctacta tgt 23
<210> 25
   <211> 20
   <212> DNA
   <213> artificial sequence
<220>
   <221> primer_bind
   <223> gene-specific primer used for 5'-RACE for *Zoanthus sp*.
<400> 25
   gtctactatg tcttgaggat 20
<210> 26
   <211> 19
   <212> DNA
   <213> artificial sequence
<220>
   <221> primer_bind
   <223> gene-specific primer used for 5'-RACE for *Discosoma sp.* "red"
<400> 26
   caagcaaatg gcaaaggtc 19
<210> 27
   <211> 19
   <212> DNA
   <213> artificial sequence
<220>
   <221> primer_bind
   <223> gene-specific primer used for 5'-RACE for *Discosoma sp.* "red"
<400> 27
   cggtattgtg gccttcgta 19
<210> 28
   <211> 19
   <212> DNA
   <213> artificial sequence
<220>
   <221> primer_bind
   <223> gene-specific primer used for 5'-RACE for *Discosoma striata*
<400> 28
   ttgtcttctt ctgcacaac 19
<210> 29
   <211> 17
   <212> DNA
   <213> artificial sequence
<220>
   <221> primer_bind
   <223> gene-specific primer used for 5'-RACE for *Discosoma striata*
<400> 29
   ctgcacaacg ggtccat 17
<210> 30
   <211> 20
   <212> DNA
   <213> artificial sequence
<220>
   <221> primer_bind
   <223> gene-specific primer used for 5'-RACE for *Anemonia sulcata*
<400> 30
   cctctatctt catttcctgc 20
<210> 31
   <211> 20
   <212> DNA
   <213> artificial sequence
<220>
   <221> primer_bind
   <223> gene-specific primer used for 5'-RACE for *Anemonia sulcata*
<400> 31
   tatcttcatt tcctgcgtac 20
<210> 32
   <211> 19
   <212> DNA
   <213> artificial sequence
<220>
   <221> primer_bind
   <223> gene-specific primer used for 5'-RACE for *Discosoma sp. "magenta"*
<400> 32
   ttcagcaccc catcacgag 19
<210> 33
   <211> 19
   <212> DNA
   <213> artificial sequence
<220>
   <221> primer_bind
   <223> gene-specific primer used for 5'-RACE for *Discosoma sp. "magenta"*
<400> 33
   acgctcagag ctgggttcc 19
<210> 34
   <211> 22
   <212> DNA
   <213> artificial sequence
<220>
   <221> primer_bind
   <223> gene-specific primer used for 5'-RACE for *Discosoma sp.* "*green*"
<400> 34
   ccctcagcaa tccatcacgt tc 22
<210> 35
   <211> 20
   <212> DNA
   <213> artificial sequence
<220>
   <221> primer_bind
   <223> gene-specific primer used for 5'-RACE for *Discosoma sp. "green"*
<400> 35
   attatctcag tggatggttc 20
<210> 36
   <211> 31
   <212> DNA
   <213> artificial sequence
<220>
   <221> primer_bind
   <223> upstream primer used to obtain full coding region of nFPs from *Anemonia majano*
<400> 36
   acatggatcc gctctttcaa acaagtttat c 31
<210> 37
   <211> 34
   <212> DNA
   <213> artificial sequence
<220>
   <221> primer_bind
   <223> downstream primer used to obtain full coding region of nFPs from *Anemonia majano*
<400> 37
   tagtactcga gcttattcgt atttcagtga aatc 34
<210> 38
   <211> 29
   <212> DNA
   <213> artificial sequence
<220>
   <221> primer_bind
   <223> upstream primer used to obtain full coding region of nFPs from *Clavularia sp.*
<400> 38
   acatggatcc aacatttttt tgagaaacg 29
<210> 39
   <211> 28
   <212> DNA
   <213> artificial sequence
<220>
   <221> primer_bind
   <223> upstream primer used to obtain full coding region of nFPs from *Clavularia sp.*
<400> 39
   acatggatcc aaagctctaa ccaccatg 28
<210> 40
   <211> 31
   <212> DNA
   <213> artificial sequence
<220>
   <221> primer_bind
   <223> downstream primer used to obtain full coding region of nFPs from *Clavularia sp.*
<400> 40
   tagtactcga gcaacacaaa ccctcagaca a 31
<210> 41
   <211> 28
   <212> DNA
   <213> artificial sequence
<220>
   <221> primer_bind
   <223> upstream primer used to obtain full coding region of nFPs from *Zoanthus sp.*
<400> 41
   acatggatcc gctcagtcaa agcacggt 28
<210> 42
   <211> 32
   <212> DNA
   <213> artificial sequence
<220>
   <221> primer_bind
   <223> downstream primer used to obtain full coding region of nFPs from *Zoanthus sp.*
<400> 42
   tagtactcga ggttggaact acattcttat ca 32
<210> 43
   <211> 31
   <212> DNA
   <213> artificial sequence
<220>
   <221> primer_bind
   <223> upstream primer used to obtain full coding region of nFPs from *Discosoma sp.* "red"
<400> 43
   acatggatcc aggtcttcca agaatgttat c 31
<210> 44
   <211> 29
   <212> DNA
   <213> artificial sequence
<220>
   <221> primer_bind
   <223> downstream primer used to obtain full coding region of nFPs from *Discosoma sp.* "red"
<400> 44
   tagtactcga ggagccaagt tcagcctta 29
<210> 45
   <211> 28
   <212> DNA
   <213> artificial sequence
<220>
   <221> primer_bind
   <223> upstream primer used to obtain full coding region of nFPs from *Discosoma striata*
<400> 45
   acatggatcc agttggtcca agagtgtg 28
<210> 46
   <211> 28
   <212> DNA
   <213> artificial sequence
<220>
   <221> primer_bind
   <223> downstream primer used to obtain full coding region of nFPs from *Discosoma striata*
<400> 46
   tagcgagctc tatcatgcct cgtcacct 28
<210> 47
   <211> 31
   <212> DNA
   <213> artificial sequence
<220>
   <221> primer-bind
   <223> upstream primer used to obtain full coding region of nFPs from *Anemonia sulcata*
<400> 47
   acatggatcc gcttcctttt taaagaagac t 31
<210> 48
   <211> 28
   <212> DNA
   <213> artificial sequence
<220>
   <221> primer_bind
   <223> downstream primer used to obtain full coding region of nFPs from *Anemonia sulcata*
<400> 48
   tagtactcga gtccttggga gcggcttg 28
<210> 49
   <211> 30
   <212> DNA
   <213> artificial sequence
<220>
   <221> primer_bind
   <223> upstream primer used to obtain full coding region of nFPs from *Discosoma sp. "magenta"*
<400> 49
   acatggatcc agttgttcca agaatgtgat 30
<210> 50
   <211> 26
   <212> DNA
   <213> artificial sequence
<220>
   <221> primer_bind
   <223> downstream primer used to obtain full coding region of nFPs from *Discosoma sp. "magenta"*
<400> 50
   tagtactcga ggccattacg ctaatc 26
<210> 51
   <211> 31
   <212> DNA
   <213> artificial sequence
<220>
   <221> primer_bind
   <223> upstream primer used to obtain full coding region of nFPs from *Discosoma sp. "green"*
<400> 51
   acatggatcc agtgcactta aagaagaaat g 31
<210> 52
   <211> 29
   <212> DNA
   <213> artificial sequence
<220>
   <221> primer_bind
   <223> downstream primer used to obtain full coding region of nFPs from *Discosoma sp. "green"*
<400> 52
   tagtactcga gattcggttt aatgccttg 29
<210> 53
   <211> 33
   <212> DNA
   <213> artificial sequence
<220>
   <221> primer_bind
   <223> TS-oligo used in cDNA synthesis and RACE
<400> 53
   aagcagtggt atcaacgcag agtacgcrgr grg 33
<210> 54
   <211> 238
   <212> PRT
   <213> *Aequorea victoria*
   <220>
   <223> amino acid sequence of GFP
<400> 54
   Met Ser Lys Gly Glu Glu Leu Phe Thr Gly Val Val Pro Ile Leu
                   5                   10                  15
   Val Glu Leu Asp Gly Asp Val Asn Gly His Lys Phe Ser Val Ser
                   20                  25                  30
   Gly Glu Gly Glu Gly Asp Ala Thr Tyr Gly Lys Leu Thr Leu Lys
                   35                  40                  45
   Phe Ile Cys Thr Thr Gly Lys Leu Pro Val Pro Trp Pro Thr Leu
                   50                  55                  60
   Val Thr Thr Phe Ser Tyr Gly Val Gln Cys Phe Ser Arg Tyr Pro
   65 70 75
   Asp His Met Lys Gln His Asp Phe Phe Lys Ser Ala Met Pro Glu
   80 85 90
   Gly Tyr Val Gln Glu Arg Thr Ile Phe Phe Lys Asp Asp Gly Asn
   95 100 105
   Tyr Lys Thr Arg Ala Glu Val Lys Phe Glu Gly Asp Thr Leu Val
   110 115 120
   Asn Arg Ile Glu Leu Lys Gly Ile Asp Phe Lys Glu Asp Gly Asn
   125 130 135
   Ile Leu Gly His Lys Leu Glu Tyr Asn Tyr Asn Ser His Asn Val
   140 145 150
   Tyr Ile Met Ala Asp Lys Gln Lys Asn Gly Ile Lys Val Asn Phe
   155 160 165
   Lys Ile Arg His Asn Ile Glu Asp Gly Ser Val Gln Leu Ala Asp
   170 175 180
   His Tyr Gln Gln Asn Thr Pro Ile Gly Asp Gly Pro Val Leu Leu
   185 190 195
   Pro Asp Asn His Tyr Leu Ser Thr Gln Ser Ala Leu Ser Lys Asp
   200 205 210
   Pro Asn Glu Lys Arg Asp His Met Val Leu Leu Glu Phe Val Thr
   215 220 225
   Ala Ala Gly Ile Thr His Gly Met Asp Glu Leu Tyr Lys
   230 235
<210> 55
   <211> 229
   <212> PRT
   <213> *Anemonia majano*
<220>
   <223> amino acid sequence of amFP486
<400> 55
   Met Ala Leu Ser Asn Lys Phe Ile Gly Asp Asp Met Lys Met Thr
   5 10 15
   Tyr His Met Asp Gly Cys Val Asn Gly His Tyr Phe Thr Val Lys
   20 25 30
   Gly Glu Gly Asn Gly Lys Pro Tyr Glu Gly Thr Gln Thr Ser Thr
   35 40 45
   Phe Lys Val Thr Met Ala Asn Gly Gly Pro Leu Ala Phe Ser Phe
   50 55 60
   Asp Ile Leu Ser Thr Val Phe Lys Tyr Gly Asn Arg Cys Phe Thr
   65 70 75
   Ala Tyr Pro Thr Ser Met Pro Asp Tyr Phe Lys Gln Ala Phe Pro
   80 85 90
   Asp Gly Met Ser Tyr Glu Arg Thr Phe Thr Tyr Glu Asp Gly Gly
   95 100 105
   Val Ala Thr Ala Ser Trp Glu Ile Ser Leu Lys Gly Asn Cys Phe
   110 115 120
   Glu His Lys Ser Thr Phe His Gly Val Asn Phe Pro Ala Asp Gly
   125 130 135
   Pro Val Met Ala Lys Lys Thr Thr Gly Trp Asp Pro Ser Phe Glu
   140 145 150
   Lys Met Thr Val Cys Asp Gly Ile Leu Lys Gly Asp Val Thr Ala
   155 160 165
   Phe Leu Met Leu Gln Gly Gly Gly Asn Tyr Arg Cys Gln Phe His
   170 175 180
   Thr Ser Tyr Lys Thr Lys Lys Pro Val Thr Met Pro Pro Asn His
   185 190 195
   Val Val Glu His Arg Ile Ala Arg Thr Asp Leu Asp Lys Gly Gly
   200 205 210
   Asn Ser Val Gln Leu Thr Glu His Ala Val Ala His Ile Thr Ser
   215 220 225
   Val Val Pro Phe
<210> 56
   <211> 266
   <212> PRT
   <213> *Clavularia sp.*
<220>
   <223> amino acid sequence of cFP484
<400> 56
   Met Lys Cys Lys Phe Val Phe Cys Leu Ser Phe Leu Val Leu Ala
   5 10 15
   Ile Thr Asn Ala Asn Ile Phe Leu Arg Asn Glu Ala Asp Phe Glu
                       20                      25                      30
   Glu Lys Thr Phe Arg Ile Pro Lys Ala Leu Thr Thr Met Gly Val
                       35                      40                      45
   Ile Lys Pro Asp Met Lys Ile Lys Leu Lys Met Glu Gly Asn Val
                       50                      55                      60
   Asn Gly His Ala Phe Val Ile Glu Gly Glu Gly Glu Gly Lys Pro
                       65                      70                      75
   Tyr Asp Gly Thr His Thr Leu Asn Leu Glu Val Lys Glu Gly Ala
                       80                      85                      90
   Pro Leu Pro Phe Ser Tyr Asp Ile Leu Ser Asn Ala Phe Gln Tyr
                       95                      100                     105
   Gly Asn Arg Ala Leu Thr Lys Tyr Pro Asp Asp Ile Ala Asp Tyr
                       110                     115                     120
   Phe Lys Gln Ser Phe Pro Glu Gly Tyr Ser Trp Glu Arg Thr Met
                       125                     130                     135
   Thr Phe Glu Asp Lys Gly Ile Val Lys Val Lys Ser Asp Ile Ser
                       140                     145                     150
   Met Glu Glu Asp Ser Phe Ile Tyr Glu Ile Arg Phe Asp Gly Met
                       155                     160                     165
   Asp Phe Pro Pro Asn Gly Pro Val Met Gln Lys Lys Thr Leu Lys
                       170                     175                     180
   Trp Glu Pro Ser Thr Glu Ile Met Tyr Val Arg Asp Gly Val Leu
                       185                     190                     195
   Val Gly Asp Ile Ser His Ser Leu Leu Leu Glu Gly Gly Gly His
                       200                     205                     210
   Tyr Arg Cys Asp Phe Lys Ser Ile Tyr Lys Ala Lys Lys Val Val
                       215                     220                     225
   Lys Leu Pro Asp Tyr His Phe Val Asp His Arg Ile Glu Ile Leu
                       230                     235                     240
   Asn His Asp Lys Asp Tyr Asn Lys Val Thr Leu Tyr Glu Asn Ala
                       245                     250                     255
   Val Ala Arg Tyr Ser Leu Leu Pro Ser Gln Ala
                       260                     265
<210> 57
   <211> 230
   <212> PRT
   <213> *Zoanthus sp.*
<220>
   <223> amino acid sequence of zFP506
<400> 57
   Ala Gln Ser Lys His Gly Leu Thr Lys Glu Met Thr Met Lys Tyr
   5 10 15
   Arg Met Glu Gly Cys Val Asp Gly His Lys Phe Val Ile Thr Gly
   20 25 30
   Glu Gly Ile Gly Tyr Pro Phe Lys Gly Lys Gln Ala Ile Asn Leu
   35 40 45
   Cys Val Val Glu Gly Gly Pro Leu Pro Phe Ala Glu Asp Ile Leu
   50 55 60
   Ser Ala Ala Phe Asn Tyr Gly Asn Arg Val Phe Thr Glu Tyr Pro
   65 70 75
   Gln Asp Ile Val Asp Tyr Phe Lys Asn Ser Cys Pro Ala Gly Tyr
   80 85 90
   Thr Trp Asp Arg Ser Phe Leu Phe Glu Asp Gly Ala Val Cys Ile
   95 100 105
   Cys Asn Ala Asp Ile Thr Val Ser Val Glu Glu Asn Cys Met Tyr
   110 115 120
   His Glu Ser Lys Phe Tyr Gly Val Asn Phe Pro Ala Asp Gly Pro
   125 130 135
   Val Met Lys Lys Met Thr Asp Asn Trp Glu Pro Ser Cys Glu Lys
   140 145 150
   Ile Ile Pro Val Pro Lys Gln Gly Ile Leu Lys Gly Asp Val Ser
   155 160 165
   Met Tyr Leu Leu Leu Lys Asp Gly Gly Arg Leu Arg Cys Gln Phe
   170 175 180
   Asp Thr Val Tyr Lys Ala Lys Ser Val Pro Arg Lys Met Pro Asp
   185 190 195
   Trp His Phe Ile Gln His Lys Leu Thr Arg Glu Asp Arg Ser Asp
   200 205 210
   Ala Lys Asn Gln Lys Trp His Leu Thr Glu His Ala Ile Ala Ser
   215 220 225
   Gly Ser Ala Leu Pro
   230
<210> 58
   <211> 230
   <212> PRT
   <213> *Zoanthus sp.*
<220>
   <223> amino acid sequence of zFP538
<400> 58
   Met Ala His Ser Lys His Gly Leu Lys Glu Glu Met Thr Met Lys
                   5                   10                  15
   Tyr His Met Glu Gly Cys Val Asn Gly His Lys Phe Val Ile Thr
                   20                  25                  30
   Gly Glu Gly Ile Gly Tyr Pro Phe Lys Gly Lys Gln Thr Ile Asn
                   35                  40                  45
   Leu Cys Val Ile Glu Gly Gly Pro Leu Pro Phe Ser Glu Asp Ile
                   50                  55                  60
   Leu Ser Ala Gly Phe Lys Tyr Gly Asp Arg Ile Phe Thr Glu Tyr
                   65                  70                  75
   Pro Gln Asp Ile Val Asp Tyr Phe Lys Asn Ser Cys Pro Ala Gly
                   80                  85                  90
   Tyr Thr Trp Gly Ser Phe Leu Phe Glu Asp Gly Ala Val Cys Ile
                   95                  100                 105
   Cys Asn Val Asp Ile Thr Val Ser Val Lys Glu Asn Cys Ile Tyr
                   110                 115                 120
   His Lys Ser Ile Phe Asn Gly Met Asn Phe Pro Ala Asp Gly Pro
                   125                 130                 135
   Val Met Lys Lys Met Thr Thr Asn Trp Glu Ala Ser Cys Glu Lys
                   140                 145                 150
   Ile Met Pro Val Pro Lys Gln Gly Ile Leu Lys Gly Asp Val Ser
                   155                 160                 165
   Met Tyr Leu Leu Leu Lys Asp Gly Gly Arg Tyr Arg Cys Gln Phe
                   170                 175                 180
   Asp Thr Val Tyr Lys Ala Lys Ser Val Pro Ser Lys Met Pro Glu
                   185                 190                 195
   Trp His Phe Ile Gln His Lys Leu Leu Arg Glu Asp Arg Ser Asp
                   200                 205                 210
   Ala Lys Asn Gln Lys Trp Gln Leu Thr Glu His Ala Ile Ala Phe
                   215                 220                 225
   Pro Ser Ala Leu Ala
                   230
<210> 59
   <211> 232
   <212> PRT
   <213> *Discosoma striata*
<220>
   <223> amino acid sequence of dsFP483
<400> 59
   Met Ser Cys Ser Lys Ser Val Ile Lys Glu Glu Met Leu Ile Asp
                   5                   10                  15
   Leu His Leu Glu Gly Thr Phe Asn Gly His Tyr Phe Glu Ile Lys
                   20                  25                  30
   Gly Lys Gly Lys Gly Gln Pro Asn Glu Gly Thr Asn Thr Val Thr
                   35                  40                  45
   Leu Glu Val Thr Lys Gly Gly Pro Leu Pro Phe Gly Trp His Ile
                   50                  55                  60
   Leu Cys Pro Gln Phe Gln Tyr Gly Asn Lys Ala Phe Val His His
                   65                  70                  75
   Pro Asp Asn Ile His Asp Tyr Leu Lys Leu Ser Phe Pro Glu Gly
                   80                  85                  90
   Tyr Thr Trp Glu Arg Ser Met His Phe Glu Asp Gly Gly Leu Cys
                   95                  100                 105
   Cys Ile Thr Asn Asp Ile Ser Leu Thr Gly Asn Cys Phe Tyr Tyr
                   110                 115                 120
   Asp Ile Lys Phe Thr Gly Leu Asn Phe Pro Pro Asn Gly Pro Val
                   125                 130                 135
   Val Gln Lys Lys Thr Thr Gly Trp Glu Pro Ser Thr Glu Arg Leu
                   140                 145                 150
   Tyr Pro Arg Asp Gly Val Leu Ile Gly Asp Ile His His Ala Leu
                   155                 160                 165
   Thr Val Glu Gly Gly Gly His Tyr Ala Cys Asp Ile Lys Thr Val
                   170                 175                 180
   Tyr Arg Ala Lys Lys Ala Ala Leu Lys Met Pro Gly Tyr His Tyr
                   185                 190                 195
   Val Asp Thr Lys Leu Val Ile Trp Asn Asn Asp Lys Glu Phe Met
   200 205 210
   Lys Val Glu Glu His Glu Ile Ala Val Ala Arg His His Pro Phe
   215 220 225
   Tyr Glu Pro Lys Lys Asp Lys
   230
<210> 60
   <211> 225
   <212> PRT
   <213> *Discosoma sp.* "red"
<220>
   <223> amino acid sequence of drFP583
<400> 60
   Met Arg Ser Ser Lys Asn Val Ile Lys Glu Phe Met Arg Phe Lys
   5 10 15
   Val Arg Met Glu Gly Thr Val Asn Gly His Glu Phe Glu Ile Glu
   20 25 30
   Gly Glu Gly Glu Gly Arg Pro Tyr Glu Gly His Asn Thr Val Lys
   35 40 45
   Leu Lys Val Thr Lys Gly Gly Pro Leu Pro Phe Ala Trp Asp Ile
   50 55 60
   Leu Ser Pro Gln Phe Gln Tyr Gly Ser Lys Val Tyr Val Lys His
   65 70 75
   Pro Ala Asp Ile Pro Asp Tyr Lys Lys Leu Ser Phe Pro Glu Gly
   80 85 90
   Phe Lys Trp Glu Arg Val Met Asn Phe Glu Asp Gly Gly Val Val
   95 100 105
   Thr Val Thr Gln Asp Ser Ser Leu Gln Asp Gly Cys Phe Ile Tyr
   110 115 120
   Lys Val Lys Phe Ile Gly Val Asn Phe Pro Ser Asp Gly Pro Val
   125 130 135
   Met Gln Lys Lys Thr Met Gly Trp Glu Ala Ser Thr Glu Arg Leu
   140 145 150
   Tyr Pro Arg Asp Gly Val Leu Lys Gly Glu Ile His Lys Ala Leu
   155 160 165
   Lys Leu Lys Asp Gly Gly His Tyr Leu Val Glu Phe Lys Ser Ile
   170 175 180
   Tyr Met Ala Lys Lys Pro Val Gln Leu Pro Gly Tyr Tyr Tyr Val
   185 190 195
   Asp Ser Lys Leu Asp Ile Thr Ser His Asn Glu Asp Tyr Thr Ile
   200 205 210
   Val Glu Gln Tyr Glu Arg Thr Glu Gly Arg His His Leu Phe Leu
   215 220 225
<210> 61
   <211> 232
   <212> PRT
   <213> *Anemonia sulcata*
<220>
   <223> amino acid sequence of asFP600
<400> 61
   Met Ala Ser Phe Leu Lys Lys Thr Met Pro Phe Lys Thr Thr Ile
   5 10 15
   Glu Gly Thr Val Asn Gly His Tyr Phe Lys Cys Thr Gly Lys Gly
   20 25 30
   Glu Gly Asn Pro Phe Glu Gly Thr Gln Glu Met Lys Ile Glu Val
   35 40 45
   Ile Glu Gly Gly Pro Leu Pro Phe Ala Phe His Ile Leu Ser Thr
   50 55 60
   Ser Cys Met Tyr Gly Ser Lys Thr Phe Ile Lys Tyr Val Ser Gly
   65 70 75
   Ile Pro Asp Tyr Phe Lys Gln Ser Phe Pro Glu Gly Phe Thr Trp
   80 85 90
   Glu Arg Thr Thr Thr Tyr Glu Asp Gly Gly Phe Leu Thr Ala His
   95 100 105
   Gln Asp Thr Ser Leu Asp Gly Asp Cys Leu Val Tyr Lys Val Lys
   110 115 120
   Ile Leu Gly Asn Asn Phe Pro Ala Asp Gly Pro Val Met Gln Asn
   125 130 135
   Lys Ala Gly Arg Trp Glu Pro Ala Thr Glu Ile Val Tyr Glu Val
   140 145 150
   Asp Gly Val Leu Arg Gly Gln Ser Leu Met Ala Leu Lys Cys Pro
   155 160 165
   Gly Gly Arg His Leu Thr Cys His Leu His Thr Thr Tyr Arg Ser
   170 175 180
   Lys Lys Pro Ala Ser Ala Leu Lys Met Pro Gly Phe His Phe Glu
   185 190 195
   Asp His Arg Ile Glu Ile Met Glu Glu Val Glu Lys Gly Lys Cys
   200 205 210
   Tyr Lys Gln Tyr Glu Ala Ala Val Gly Arg Tyr Cys Asp Ala Ala
   215 220 225
   Pro Ser Lys Leu Gly His Asn
   230
<210> 62
   <211> 231
   <212> PRT
   <213> *Discosoma sp. "green"*
<220>
   <223> amino acid sequence of dgFP512
<400> 62
   Met Ser Ala Leu Lys Glu Glu Met Lys Ile Asn Leu Thr Met Glu
   5 10 15
   Gly Val Val Asn Gly Leu Pro Phe Lys Ile Arg Gly Asp Gly Lys
   20 25 30
   Gly Lys Pro Tyr Gln Gly Ser Gln Glu Leu Thr Leu Thr Val Val
   35 40 45
   Lys Gly Gly Pro Leu Pro Phe Ser Tyr Asp Ile Leu Thr Thr Met
   50 55 60
   Phe Gln Tyr Gly Asn Arg Ala Phe Val Asn Tyr Pro Glu Asp Ile
   65 70 75
   Pro Asp Ile Phe Lys Gln Thr Cys Ser Gly Pro Asn Gly Gly Tyr
   80 85 90
   Ser Trp Gln Arg Thr Met Thr Tyr Glu Asp Gly Gly Val Cys Thr
   95 100 105
   Ala Thr Ser Asn Ile Ser Val Val Gly Asp Thr Phe Asn Tyr Asp
   110 115 120
   Ile His Phe Met Gly Ala Asn Phe Pro Leu Asp Gly Pro Val Met
                   125                 130                 135
   Gln Lys Arg Thr Met Lys Trp Glu Pro Ser Thr Glu Ile Met Phe
                   140                 145                 150
   Glu Arg Asp Gly Met Leu Arg Gly Asp Ile Ala Met Ser Leu Leu
                   155                 160                 165
   Leu Lys Gly Gly Gly His Tyr Arg Cys Asp Phe Glu Thr Ile Tyr
                   170                 175                 180
   Lys Pro Asn Lys Val Val Lys Met Pro Asp Tyr His Phe Val Asp
                   185                 190                 195
   His Cys Ile Glu Ile Thr Ser Gln Gln Asp Tyr Tyr Asn Val Val
                   200                 205                 210
   Glu Leu Thr Glu Val Ala Glu Ala Arg Tyr Ser Ser Leu Glu Lys
                   215                 220                 225
   Ile Gly Lys Ser Lys Ala
                   230
<210> 63
   <211> 235
   <212> PRT
   <213> *Discosoma sp. "magenta"*
<220>
   <223> amino acid sequence of dmFP592
<400> 63

## Description

### BACKGROUND OF THE INVENTION

### Field of the Invention

This invention relates to the field of molecular biology. More specifically, this invention relates to novel fluorescent proteins from non-bioluminescent species of class anthozoa, methods of identifying the DNA sequences encoding the proteins, genes encoding such proteins and uses thereof.

### Description of the Related Art

Fluorescence labeling is a particularly useful tool for marking a protein, cell, or organism of interest. Traditionally, a protein of interest is purified, then covalently conjugated to a fluorophore derivative. For *in vivo* studies, the protein-dye complex is then inserted into cells of interest using micropipetting or a method of reversible permeabilization. The dye attachment and insertion steps, however, make the process laborious and difficult to control. An alternative method of labeling proteins of interest is to concatenate or r fuse the gene expressing the protein of interest to a gene expressing a marker, then express the fusion product. Typical markers for this method of protein labeling include β-galactosidase, firefly luciferase and bacterial luciferase. These markers, however, require exogenous substrates or cofactors and are therefore of limited use for *in vivo* studies.

A marker that does not require an exogenous cofactor or substrate is the green fluorescent protein (GFP) of the jellyfish *Aequorea victoria,* a protein with an excitation maximum at 395 nm, a second excitation peak at 475 nm and an emission maximum at 510 nm. GFP is a 238-amino acid protein, with amino acids 65-67 involved in the formation of the chromophore.

Uses of GFP for the study of gene expression and protein localization are discussed in detail by Chalfie et al. in Science 263 (1994), 802-805, and Heim et al. in Proc. Nat. Acad. Sci. 91 (1994), 12501-12504. Additionally, Rizzuto et al. in Curr. Biology 5 (1995), 635-642, discuss the use of wild-type GFP as a tool for visualizing subcellular organelles in cells, while Kaether and Gerdes in Febs Letters 369 (1995), 267-271, report the visualization of protein transport along the secretory pathway using wild-type GFP. The expression of GFP in plant cells is discussed by Hu and Cheng in Febs Letters 369 (1995), 331-334, while GFP expression in *Drosophila* embryos is described by Davis et al. in Dev. Biology 170 (1995), 726-729.

Crystallographic structures of wild-type GFP and the mutant GFP S65T reveal that the GFP tertiary structure resembles a barrel (Ormö et al., Science 273 (1996), 1392-1395; Yang, et al., Nature Biotechnol 14 (1996), 1246-1251). The barrel consists of beta sheets in a compact structure, where, in the center, an alpha helix containing the chromophore is shielded by the barrel. The compact structure makes GFP very stable under diverse and/or harsh conditions such as protease treatment, making GFP an extremely useful reporter in general. However, the stability of GFP makes it sub-optimal for determining short-term or repetitive events.

A great deal of research is being performed to improve the properties of GFP and to produce GFP reagents useful and optimized fro a variety of research purposes. New versions of GFP have been developed, such as a "humanized" GFP DNA, the protein product of which has increased synthesis in mammalian cells (Haas, et al., Current Biology 8 (1996), 315-324; Yang, et al., Nucleic Acids Research 24 (1996), 4592-4593). One such humanized protein is "enhanced green fluorescent protein" (EGFP). Other mutations to GFP have resulted in blue-, cyan- and yellow-green light emitting versions. Despite the great utility of GFP, however, other fluorescent proteins with properties similar to or different from GFP would be useful in the art. Novel fluorescent proteins result In possible new colors, or produce pH-dependent fluorescence. Other benefits of novel fluorescent proteins include fluorescence resonance energy transfer (FERT) possibilities based on new spectra and better suitability for larger excitation.

Jörg Wiedenmann in his diploma thesis "Zur Biologie der Öketypen von *Anemonia sulcata* (Pennant)" 1996 describes his attempts to categorise *Anemonia sulcata* according to its environment. In doing this, the author examines the habitat, tentacle colour, genotype and behavioural aggressiveness. Upon examination of tentacle colour, the author determines that this is partly due to Zooxanthellae and partly due to the anemone's own colours, which the author describes as being genetically fixed fluorescent proteins.

The prior art is deficient in novel fluorescent proteins wherein the DNA coding sequences are known. The present invention fulfils this long-standing need in the art.

### SUMMARY OF THE INVENTION

The present invention is directed to a nucleic acid in other than its natural environment, the sequence of which encodes a fluorescent protein, wherein said fluorescent protein is a protein from a non-bioluminescent Anthozoan organism selected from the genera Anemonia. Clavularia, Zoanthus and Discosoma, and wherein;
(a) said fluorescent protein is from Anemonia majano and said nucleic acid can be amplified with a pair of primers, the first of which consists of the sequence of SEQ ID NO:36 and the second of which consists of the sequence of SEQ ID NO:37; or
(b) said fluorescent protein is from Clavularia sp. and said nucleic acid can be amplified with a primer pair consisting of a first primer which consists of the sequence of SEQ ID NO:38 and a second primer which consists of the sequence of SEQ ID NO:40; or
(c) said fluorescent protein is from Zoanthus sp. and said nucleic acid can be amplified with a primer pair consisting of a first primer which consists of the sequence of SEQ ID NO:41 and a second primer which consists of the sequence of SEQ ID NO:42; or
(d) said fluorescent protein is from Discosoma sp. "red" and said nucleic acid can be amplified with a primer pair consisting of a first primer which consists of the sequence of SEQ ID NO:43 and a second primer which consists of the sequence of SEQ ID NO:44; or
(e) said fluorescent protein is from Discosoma striata and said nucleic acid can be amplified with a primer pair consisting of a first primer which consists of the sequence of SEQ ID NO:45 and a second primer which consists of the sequence of SEQ ID NO:46.

Described is a method of identifying a DNA sequence encoding a fluorescent protein comprising the step of screening for an existence of a nucleic acid sequence in a sample, wherein the nucleic acid sequence encodes a peptide having a sequence selected from the group consisting of SEQ ID Nos. 3, 5, 8, 11, 12 and 14. The existence of the nucleic acid sequence identifies the DNA sequence encoding the fluorescent protein.

Also describes is a method of identifying a DNA sequence encoding a fluorescent protein comprising the step of screening for an existence of a nucleic acid sequence in a sample, wherein the nucleic acid sequence hybridizes to a primer selected from the group consisting of SEQ ID Nos. 4, 6, 7, 9, 10, 13, 15 and 16. The existence of the nucleic acid sequence identifies the DNA sequence encoding the fluorescent protein.

Also described is a method of analyzing a fluorescent protein in a cell, comprising the steps of expressing a nucleic acid sequence encoding a fluorescent protein having an amino acid sequence selected from the group consisting of SEQ ID Nos. 55-63 in the cell; and measuring a fluorescence signal from the protein. This method further comprises a step of sorting the cell according to the signal. Preferably, the cell is sorted by fluorescence activated cell sorting. Still preferably, the nucleic acid sequence comprises a gene of interest encoding a protein of interest fused to the fluorescent protein, wherein the protein of interest is distinct from the fluorescent protein. The detected fluorescence signal indicates the presence of the gene of interest and further the protein of interest in the cell. By identifying an intracellular location of the fluorescent protein, an intracellular location of the protein of interest is also identified.

Other and further aspects, features, and advantages of the present invention will be apparent from the following description of the presently preferred embodiments of the invention given for the purpose of disclosure.

### BRIEF DESCRIPTION OF THE DRAWINGS

**Figure 1** shows the modified strategy of 3'-RACE used to isolate the target fragments. Sequences of the oligonucleotides used are shown in Table 2. Dp1 and Dp2 are the degenerate primers used in the first and second PCR, respectively (see Tables 3 and 4 for the sequences of degenerate primers).

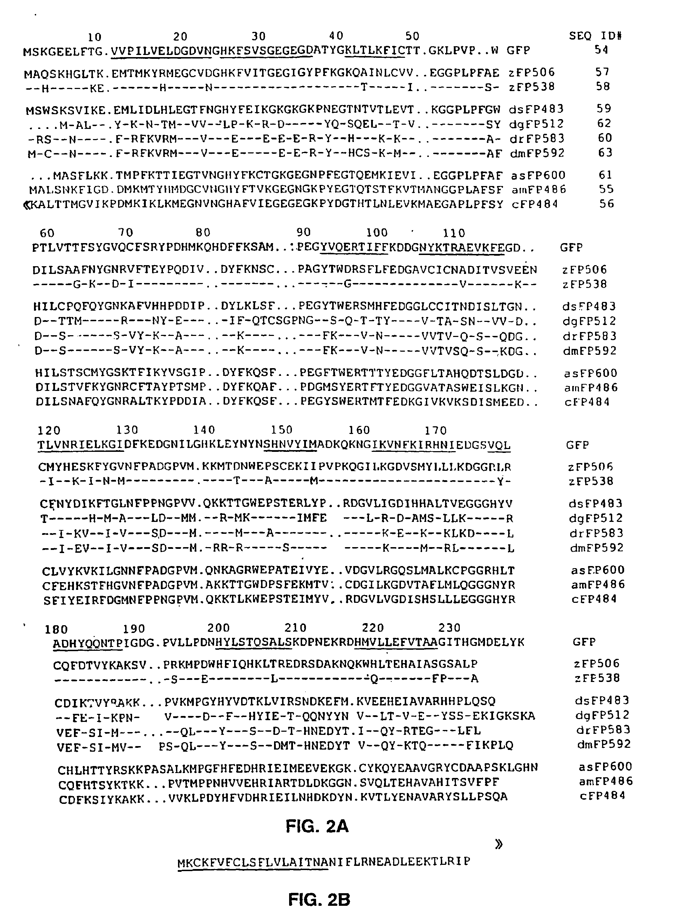

**Figure 2A** shows multiple alignment of novel fluorescent proteins. The numbering is based on *Aequorea victoria* green fluorescent protein (GFP). Two proteins from *Zoanthus* and four from *Discosoma* are compared between each other: residues identical to the corresponding ones in the first protein of the series are represented by dashes. Introduced gaps are represented by dots. In the sequence of *A. victoria* GFP, the stretches forming beta-sheets are underlined; the residues whose side chains form the interior of the beta-can are shaded (according to Yang et al., Nature Biotechnol. 14, 1246-1251 (1996). **Figure 2B** shows the N-terminal part of cFP484, which has no homology with the other proteins. The putative signal peptide is underlined.
**Figure 3** shows the excitation and emission spectrum of the novel fluorescent protein from *Anemonia majano,* amFP486.
**Figure 4** shows the excitation and emission spectrum of the novel fluorescent protein from *Clavularia,* cFP484.
**Figure 5** shows the excitation and emission spectrum of the novel fluorescent protein from *Zoanthus,* zFP506.
**Figure 6** shows the excitation and emission spectrum of the novel fluorescent protein from *Zoanthus,* zFP538.
**Figure 7** shows the excitation and emission spectrum of the novel fluorescent protein from *Discosoma striata,* dsFP483.
**Figure 8** shows the excitation and emission spectrum of the novel fluorescent protein from *Discosoma,* drFP583.
**Figure 9** shows the excitation and emission spectrum of the novel fluorescent protein from *Anemonia sulcata,* asFP600. This protein is no embodiment of the present invention.
**Figure 10** shows the excitation and emission spectrum of the novel fluorescent protein from *Discosoma,* dgFP512. This protein is no embodiment of the present invention.
**Figure 11** shows the excitation and emission spectrum of the novel fluorescent protein from *Discosoma,* dmFP592. This protein is no embodiment of the present invention.

### DETAILED DESCRIPTION OF THE INVENTION

As used herein, the term "GFP" refers to the basic green fluorescent protein from *Aequorea victoria,* including prior art versions of GFP engineered to provide greater fluorescence or fluoresce in different colors. The sequence of *Aequorea victoria* GFP (SEQ ID No. 54) has been disclosed in Prasher et al., Gene 111 (1992), 229-33.

As used herein, the term "EGFP" refers to mutant variant of GFP having two amino acid substitutions: F64L and S65T (Heim et al., Nature 373 (1995), 663-664). The term "humanized" refers to changes made to the GFP nucleic acid sequence to optimize the codons for expression of the protein in human cells (Yang et al., Nucleic Acids Research 24 (1996), 4592-4593).

In accordance with the present invention there may be employed conventional molecular biology, microbiology, and recombinant DNA techniques within the skill of the art. Such techniques are explained fully in the literature. See, e.g., Maniatis, Fritsch & Sambrook, "Molecular Cloning: A Laboratory Manual (1982); "DNA Cloning: A Practical Approach," Volumes I and II (D.N. Glover ed. 1985); "Oligonucleotide Synthesis" (M.J. Gait ed. 1984); "Nucleic Acid Hybridization" (B.D. Hames & S.J. Higgins eds. (1985)); "Transcription and Translation" (B.D. Hames & S.J. Higgins eds. (1984)); "Animal Cell Culture" (R.I. Freshney, ed. (1986)); "Immobilized Cells and Enzymes" (IRL Press, (1986)); B. Perbal, "A Practical Guide To Molecular Cloning" (1984).

A "vector" is a replicon, such as plasmid, phage or cosmid, to which another DNA segment may be attached so as to bring about the replication of the attached segment.

A "DNA molecule" refers to the polymeric form of deoxyribonucleotides (adenine, guanine, thymine, or cytosine) in either single stranded form or a double-stranded helix. This term refers only to the primary and secondary structure of the molecule, and does not limit it to any particular tertiary forms. Thus, this term includes double-stranded DNA found, inter alia, in linear DNA molecules (e.g., restriction fragments), viruses, plasmids, and chromosomes.

A DNA "coding sequence" is a DNA sequence which is transcribed and translated into a polypeptide in vivo when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and synthetic DNA sequences. A polyadenylation signal and transcription termination sequence may be located 3' to the coding sequence.

As used herein, the term "hybridization" refers to the process of association of two nucleic acid strands to form an antiparallel duplex stabilized by means of hydrogen bonding between residues of the opposite nucleic acid strands.

The term "oligonucleotide" refers to a short (under 100 bases in length) nucleic acid molecule.

"DNA regulatory sequences", as used herein, are transcriptional and translational control sequences, such as promoters, enhancers, polyadenylation signals, terminators, and the like, that provide for and/or regulate expression of a coding sequence in a host cell.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site, as well as protein binding domains responsible for the binding of RNA polymerase. Eukaryotic promoters will often, but not always, contain "TATA" boxes and "CAT" boxes. Various promoters, including inducible promoters, may be used to drive the various vectors of the present invention.

As used herein, the terms "restriction endonucleases" and "restriction enzymes" refer to bacterial enzymes, each of which cut double-stranded DNA at or near a specific nucleotide sequence.

A cell has been "transformed" or "transfected" by exogenous or heterologous DNA when such DNA has been introduced inside the cell. The transforming DNA may or may not be integrated (covalently linked) into the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or common ancestor by mitosis. A "cell line" is a clone of a primary cell that is capable of stable growth *in vitro* for many generations.

A "heterologous" region of the DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. In another example, heterologous DNA includes coding sequence in a construct where portions of genes from two different sources have been brought together so as to produce a fusion protein product. Allelic variations or naturally-occurring mutational events do not give rise to a heterologous region of DNA as defined herein.

As used herein, the term "reporter gene" refers to a coding sequence attached to heterologous promoter or enhancer elements and whose product may be assayed easily and quantifiably when the c construct is introduced into tissues or cells.

The amino acids described herein are preferred to be in the "L" isomeric form. The amino acid sequences are given in one-letter code (A: alanine; C: cysteine; D: aspartic acid; E: gluetamic acid; F: phenylalanine; G: glycine; H: histidine; I: isoleucine; K: lysine; L: leucine; M: metionine; N: asparagine; P: proline; Q: gluetamine; R: arginine; S: serine; T: threonine; V: valine; W: tryptophane; Y: tyrosine; X: any residue). NH₂ refers to the free amino group present at the amino terminus of a polypeptide. COOH refers to the free carboxy group present at the carboxy terminus of a polypeptide. In keeping with standard polypeptide nomenclature, J Biol, Chem., 243 (1969), 3552-59 is used.

The present invention is directed to an isolated and purified fluorescent protein selected from the group consisting of amFP486, cFP484, zFP506. zFP538, dsFP483, and drFP583.

Herein described is a method of identifying a DNA sequence encoding a fluorescent protein comprising the step of screening for an existence of a nucleic acid sequence in a sample, wherein the nucleic acid sequence encodes a peptide having a sequence selected from the group consisting of SEQ ID Nos. 3, 5, 8, 11, 12 and 14. The existence of the nucleic acid sequence identifies the DNA sequence encoding the fluorescent protein.

Also described is a method of identifying a DNA sequence encoding a fluorescent protein comprising the step of screening for an existence of a nucleic acid sequence in a sample, wherein the nucleic acid sequence hybridizes to a primer selected from the group consisting of SEQ ID Nos. 4, 6, 7, 9, 10, 13, 15 and 16. The existence of the nucleic acid sequence identifies the DNA sequence encoding the fluorescent protein.

Further described is a method of analyzing a fluorescent protein in a cell, comprising the steps of expressing a nucleic acid sequence encoding a fluorescent protein having an amino acid sequence selected from the group consisting of SEQ ID Nos. 55-63 in the cell; and measuring a fluorescence signal from the protein. This method further comprises a step of sorting the cell according to the signal. Preferably, the cell is sorted by fluorescence activated cell sorting. Still preferably, the nucleic acid sequence comprises a gene of interest encoding a protein of interest fused to the fluorescent protein, wherein the protein of interest is distinct from the fluorescent protein. The detected fluorescence signal indicates the presence of the gene of interest and further the protein of interest in the cell. By identifying an intracellular location of the fluorescent protein, an intracellular location of the protein of interest is also identified.

The following examples are given for the purpose of illustrating various embodiments of the invention and are not meant to limit the present invention in any fashion.

### Biological Material

Novel fluorescent proteins were identified from several genera of Anthozoa which do not exhibit any bioluminescence but have fluorescent color as observed under usual white light or ultraviolet light. Six species were chosen (see Table 1).

**TABLE 1**

| Anthozoa Species Used in This Study | | |
|---|---|---|
| Species | Area of Origination | Fluorescent Color |
| Anemonia majano | Western Pacific | bright green tentacle tips |
| Clavularia sp. | Western Pacific | bright green tentacles and oral disk |
| Zoanthus sp. | Western Pacific | green-yellow tentacles and oral disk |
| Discosoma sp. "red" | Western Pacific | orange-red spots oral disk |
| Discosoma striata | Western Pacific | blue-green stripes on oral disk |
| Discosoma sp. "magenta" | Western Pacific | faintly purple oral disk |
| Discosoma sp. "green" | Western Pacific | green spots on oral disk |
| Anemonia sulcata | Mediterranean | purple tentacle tips |

### EXAMPLE 2

### cDNA Preparation

Total RNA was isolated from the species of interest according to the protocol of Chomczynski and Sacchi (Chomczynski P., et al., Anal. Biochem. 162 (1987), 156-159). First-strand cDNA was synthetized starting with 1-3 µg of total RNA using SMART PCR cDNA synthesis kit (CLONTECH) according to the provided protocol with the only alteration being that the "cDNA synthesis primer" provided in the kit was replaced by the primer TN3 (5'- CGCAGTCGACCG(T)₁₃, SEQ ID No. 1) (Table 2). Amplified cDNA samples were then prepared as described in the protocol provided except the two primers used for PCR were the TS primer (5'-AAGCAGTGGTATCAACGCAGAGT, SEQ ID No. 2) (Table 2) and the TN3 primer (Table 2), both in 0.1 µM concentration. Twenty to twenty-five PCR cycles were performed to amplify a cDNA sample. The amplified cDNA was diluted 20-fold in water and 1 µl of this dilution was used in subsequent procedures.

**TABLE 2**

| Oligos Used in cDNA Synthesis and RACE | |
|---|---|
| TN3: | 5'-CGCAGTCGACCG(T)₁₃ |
| | (SEQ ID No. 1) |
| | |
| T7-TN3 : | 5'-GTAATACGACTCACTATAGGGCCGCAGTCGACCG(T)₁₃ |
| | (SEQ ID No. 17) |
| | |
| TS-primer: | 5'-AAGCAGTGGTATCAACGCAGAGT |
| | (SEQ ID No. 2) |
| | |
| T7-TS: | 5'-GTAATACGACTCACTATAGGGCAAGCAGTGGTATCAACGCAGAGT |
| | (SEQ ID No. 18) |
| | |
| T7: | 5'-GTAATACGACTCACTATAGGGC |
| | (SEQ ID No. 19) |
| | |
| TS-oligo | 5'-AAGCAGTGGTATCAACGCAGAGTACGCrGrGrG |
| | (SEQ ID No. 53) |
| | |

### EXAMPLE 3

### Oligo Design

To isolate fragments of novel fluorescent protein cDNAs, PCR using degenerate primers was performed. Degenerate primers were designed to match the sequence of the mRNAs in regions that were predicted to be the most invariant in the family of fluorescent proteins. Four such stretches were chosen (Table 3) and variants of degenerate primers were designed. All such primers were directed to the 3'-end of mRNA. All oligos were gel-purified before use. Table 2 shows the oligos used in cDNA synthesis and RACE.

**TABLE 3**

| Key Amino Acid Stretches and Corresponding Degenerate Primers Used for Isolation of Fluorescent Proteins | | |
|---|---|---|
| Stretch Position according to A. victoria GFP (7) | Amino Acid Sequence of the Key Stretch | Degenerated Primer Name and Sequence |
| 20-25 | GXVNGH (SEQ ID No. 3) | |
| 31-35 | GEGEG (SEQ ID No. 5) | |
| | | |
| | GEGNG (SEQ ID No. 8) | |
| | | |
| 127-131 | GMNFP (SEQ ID No. 11) | |
| | GVNFP (SEQ ID No. 12) | |
| 134-137 | GPVM (SEQ ID No. 14) | |
| | | |

### EXAMPLE 4

### Isolation of 3'-cDNA Fragments of nFPs

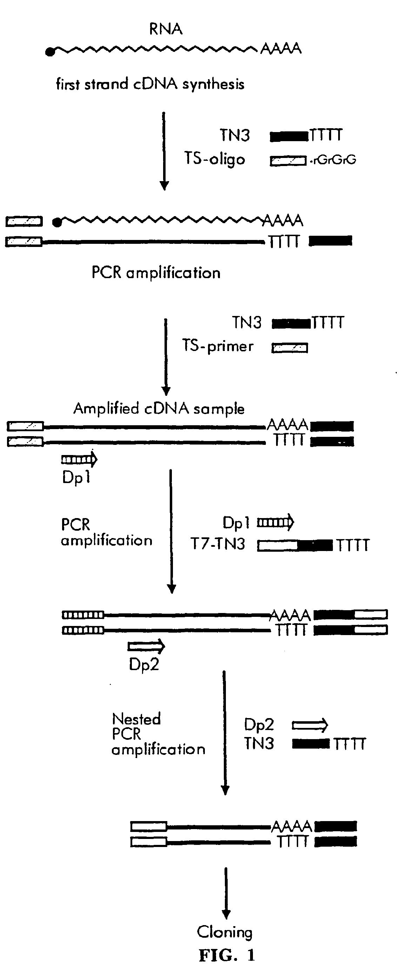

The modified strategy of 3'-RACE was used to isolate the target fragments (see Figure 1). The RACE strategy involved two consecutive PCR steps. The first PCR step involved a first degenerate primer (Table 4) and the T7-TN3 primer (SEQ ID No. 17) which has a 3' portion identical to the TN3 primer used for cDNA synthesis (for sequence of T7-TN3, Table 2). The reason for substituting the longer T7-TN3 primer in this PCR step was that background amplification which occurred when using the shorter TN3 primer was suppressed effectively, particularly when the T7-TN3 primer was used at a low concentration (0.1 _M) (Frohman et al., (1998) PNAS USA, 85, 8998-9002). The second PCR step involved the TN3 primer (SEQ ID No. 1, Table 2) and a second degenerate primer (Table 4).

**TABLE 4**

| Combinations of Degenerate Primers for First and Second PCR Resulting in Specific Amplification of 3'-Fragments of nFP cDNA | | |
|---|---|---|
| Species | First Degenerate Primer | Second Degenerate Primer |
| Anemonia majano | NGH | GNGb |
| | (SEQ ID No. 4) | (SEQ ID No. 10) |
| Clavularia sp. | NGH | GEGa |
| | (SEQ ID No. 4) | (SEQ ID No. 6) |
| Zoanthus sp. | NGH | GEGa |
| | (SEQ ID No. 4) | (SEQ ID No. 6) |
| Discosoma sp. "red" | NGH | GEGa (SEQ ID No. 6), |
| | (SEQ ID No. 4) | NFP (SEQ ID No. 13) or |
| | | PVMb (SEQ ID No. 16) |
| Discosoma striata | NGH | NFP |
| | (SEQ ID No. 4) | (SEQ ID No. 13) |
| Anemonia sulcata | NGH | GEGa (SEQ ID No. 6) |
| | (SEQ ID No. 4) | or NFP (SEQ ID No. 13) |

The first PCR reaction was performed as follows: 1 µl of 20-fold dilution of the amplified cDNA sample was added into the reaction mixture containing 1X Advantage KlenTaq Polymerase Mix with provided buffer (CLONTECH), 200 µM dNTPs, 0.3 µM of first degenerate primer (Table 4) and 0.1 µM of T7-TN3 (SEQ ID No. 17) primer in a total volume of 20 µl. The cycling profile was (Hybaid OmniGene Thermocycler, tube control mode): 1 cycle for 95°C, 10 sec.; 55°C, 1 min.; 72°C, 40 sec; 24 cycles for 95°C, 10 sec.; 62°C, 30 sec.; 72°C, 40 sec. The reaction was then diluted 20-fold in water and 1 µl of this dilution was added to a second PCR reaction, which contained 1X Advantage KlenTaq Polymerase Mix with the buffer provided by the manufacturer (CLONTECH), 200 µM dNTPs, 0.3 µM of the second degenerate primer (Table 4) and 0.1 µM of TN3 primer. The cycling profile was (Hybaid OmniGene Thermocycler, tube control mode): 1 cycle for 95°C, 10 sec.; 55°C (for GEG/GNG or PVM) or 52°C (for NFP), 1 min.; 72°C, 40 sec; 13 cycles for 95°C, 10sec.; 62°C (for GEG/GNG or PVM) or 58°C (for NFP), 30 sec.; 72°C, 40 sec. The product of PCR was cloned into PCR-Script vector (Stratagene) according to the manufacturer's protocol.

Different combinations of degenerate primers were tried in the first and second PCR reactions on the DNA from each species until a combination of primers was found that resulted in specific amplification--meaning that a pronounced band of expected size (about 650-800 bp for NGH and GEG/GNG and 350-500 bp for NFP and PVM--sometimes accompanied by a few minor bands) was detected on agarose gel after two PCR reactions. The primer combinations of choice for different species of the Class Anthozoa are listed in Table 4. Some other primer combinations also resulted in amplification of fragments of correct size, but the sequence of these fragments showed no homology to the other fluorescent proteins identified or to *Aequorea victoria* GFP.

### EXAMPLE 5

### Obtaining Full-Length cDNA Copies

Upon sequencing the obtained 3'-fragments of novel fluorescent protein cDNAs, two nested 5'-directed primers were synthesized for cDNA (Table 5), and the 5' ends of the cDNAs were then amplified using two consecutive PCRs. In the next PCR reaction, the novel approach of "step-out PCR" was used to suppress background amplification. The step-out reaction mixture contained 1x Advantage KlenTaq Polymerase Mix using buffer provided by the manufacturer (CLONTECH), 200 µM dNTPs, 0.2 µM of the first gene-specific primer (see Table 5), 0.02 µM of the T7-TS primer (SEQ ID No. 18), 0.1 µM of T7 primer (SEQ ID No. 19) and 1 µl of the 20-fold dilution of the amplified cDNA sample in a total volume of 20 µl. The cycling profile was (Hybaid OmniGene Thermocycler, tube control mode): 23-27 cycles for 95°C, 10 sec.; 60°C, 30 sec.; 72°C, 40 sec. The product of amplification was diluted 50-fold in water and one µl of this dilution was added to the second (nested) PCR. The reaction contained 1X Advantage KlenTaq Polymerase Mix with provided buffer (CLONTECH), 200 µM dNTPs, 0.2 µM of the second gene-specific primer and 0.1 µM of TS primer (SEQ ID No. 2) in a total volume of 20 µl. The cycling profile was (Hybaid OmniGene Thermocycler, tube control mode): 12 cycles for 95°C, 10 sec.; 60°C, 30 sec.; 72°C, 40 sec. The product of amplification was then cloned into pAtlas vector (CLONTECH) according to the manufacturer's protocol.

It is noted that the last 3 lines of Table 5 do not relate to embodiments of the present invention.

**TABLE 5**

| Gene-Specific Primers Used for 5'-RACE | | |
|---|---|---|
| Species | First Primer | Second (Nested) Primer |
| Anemonia majano | 5'-GAAATAGTCAGGCATACTGGT | |
| | (SEQ ID No. 20) | |
| Clavularia sp. | 5'-CTTGAAATAGTCTGCTATATC | |
| | (SEQ ID No. 22) | |
| Zoanthus sp. | | |
| Discosoma sp. "red" | 5'-CAAGCAAATGGCAAAGGTC | |
| | (SEQ ID No. 26) | |
| Discosoma striata | 5'-TTGTCTTCTTCTGCACAAC | |
| | (SEQ ID No. 28) | |
| Anemonia sulcata | 5'-CCTCTATCTTCATTTCCTGC | |
| | (SEQ ID No. 30) | |
| Discosoma sp. "magenta" | 5'-TTCAGCACCCCATCACGAG | |
| | (SEQ ID No. 32) | |
| Discosoma sp. "green" | 5'-CCCTCAGCAATCCATCACGTTC | |
| | (SEQ ID No. 34) | |

### EXAMPLE 6

### Expression of nFPs in E. co/i

To prepare a DNA construct for novel fluorescent protein expression, two primers were synthesized for each cDNA: a 5'-directed "downstream" primer with the annealing site located in the 3'-UTR of the cDNA and a 3'-directed "upstream" primer corresponding to the site of translation start site (not including the first ATG codon) (Table 6). Both primers had 5'-heels coding for a site for a restriction endonuclease; in addition, the upstream primer was designed so as to allow the cloning of the PCR product into the pQE30 vector (Qiagen) in such a way that resulted in the fusion of reading frames of the vector-encoded 6xHis-tag and nFP. The PCR was performed as follows: 1 µl of the 20-fold dilution of the amplified cDNA sample was added to a mixture containing 1x Advantage KlenTaq Polymerase Mix with buffer provided by the manufacturer (CLONTECH), 200 µM dNTPs, 0.2 µM of upstream primer and 0.2 µM of downstream primer, in a final total volume of 20 µl. The cycling profile was (Hybaid OmniGene Thermocycler, tube control mode): 23-27 cycles for 95°C, 10 sec.; 60°C, 30 sec.; 72°C, 40 sec. The product of this amplification step was purified by phenol-chlorophorm extraction and ethanol precipitation and then cloned into pQE30 vector using restriction endonucleases corresponding to the primers' sequence according to standard protocols.

All plasmids were amplified in XL-1 blue *E. coli* and purified by plasmid DNA miniprep kits (CLONTECH). The recombinant clones were selected by colony color, and grown in 3 ml of LB medium (supplemented with 100 µg/ml of ampicillin) at 37°C overnight. 100 µl of the overnight culture was transferred into 200 ml of fresh LB medium containing 100 µg/ml of ampicillin and grown at 37°C, 200 rpm up to OD₆₀₀ 0.6-0.7. 1 mM IPTG was then added to the culture and incubation was allowed to proceed at 37°C for another 16 hours. The cells were harvested and recombinant protein, which incorporated 6 x His tags on the N-terminus, was purified using TALON™ metal-affinity resin according to the manufacturer's protocol (CLONTECH).

It is noted that the last three lines of Table 6 do not relate to embodiments of the present invention.

**TABLE 6**

| Primers Used to Obtain Full Coding Region of nFPs for Cloning into Expression Construct | | |
|---|---|---|
| Species | Upstream Primer | Downstream Primer |
| Anemonia majano | | |
| Clavularia | | |
| sp. | | |
| Zoanthus sp. | | |
| Discosoma sp. "red" | | |
| Discosoma striata | | |
| Anemonia sulcata | | |
| Discosoma sp. "magenta" | | |
| Discosoma sp. "green" | | |

### EXAMPLE 7

### Novel Fluorescent Proteins_and cDNAs Encoding the Proteins

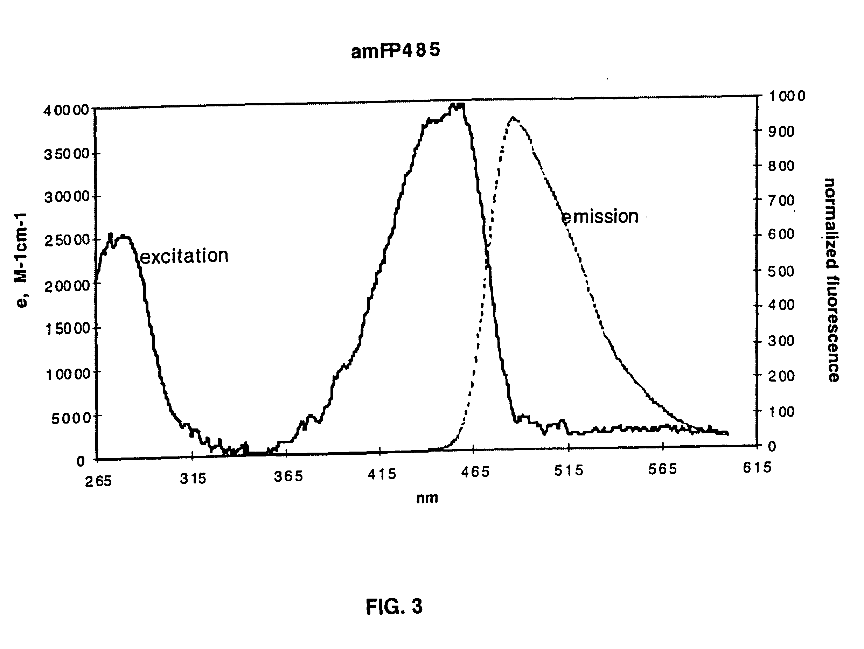

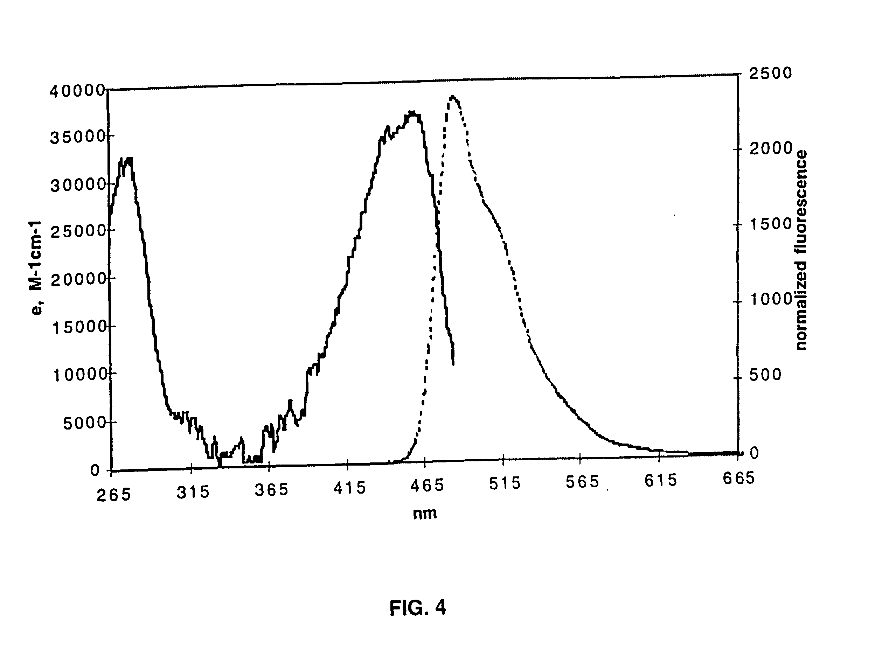

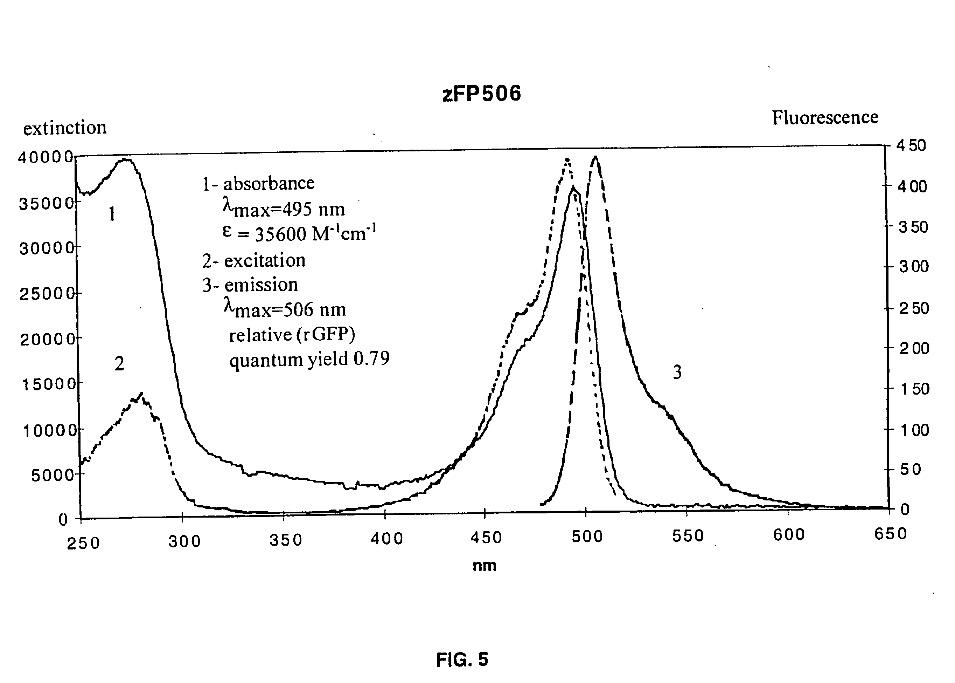

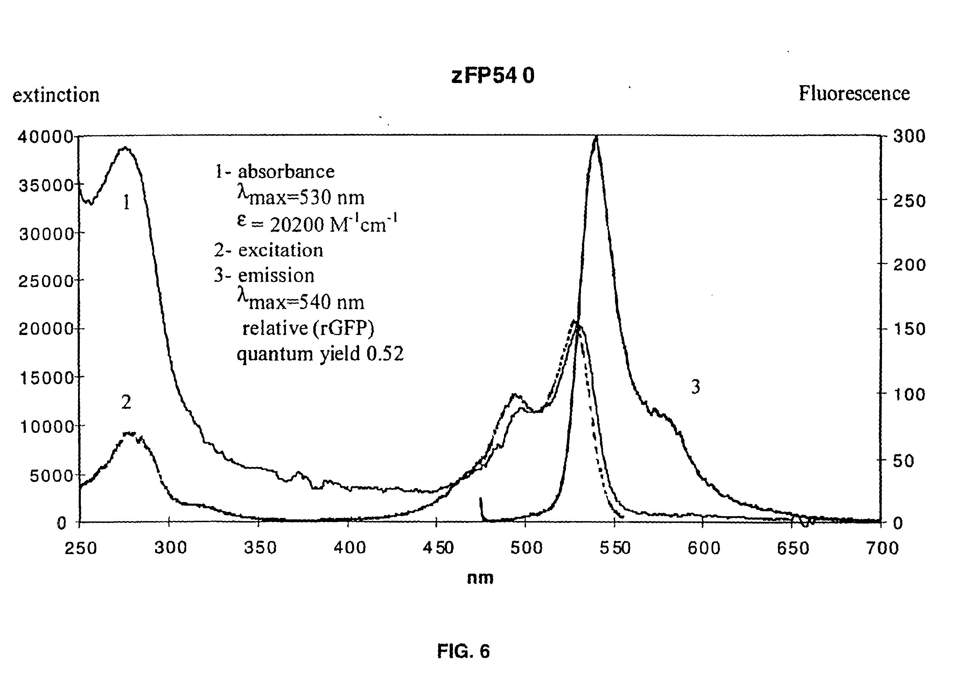

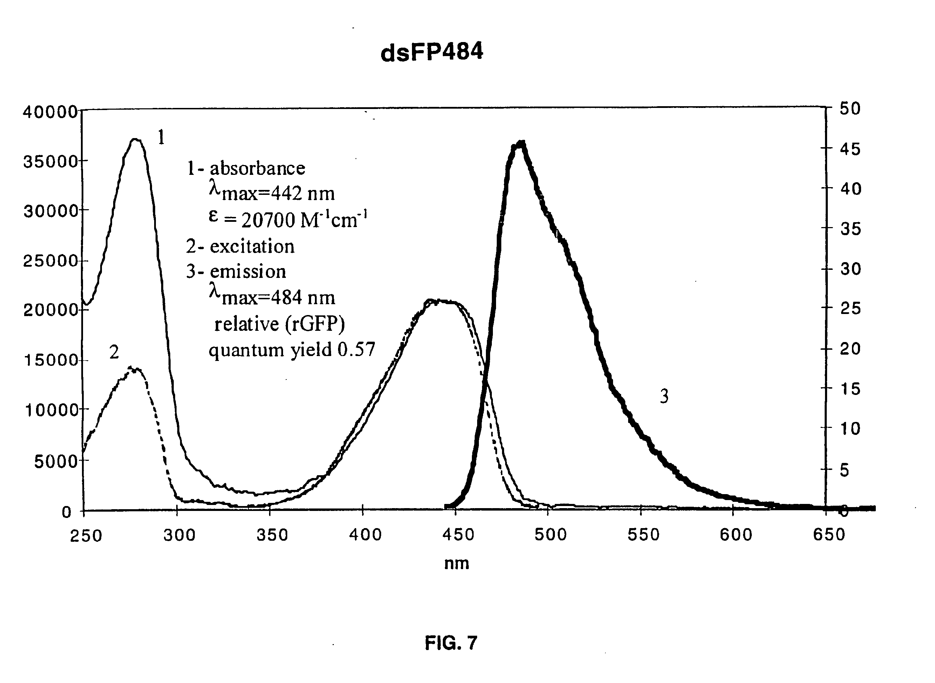

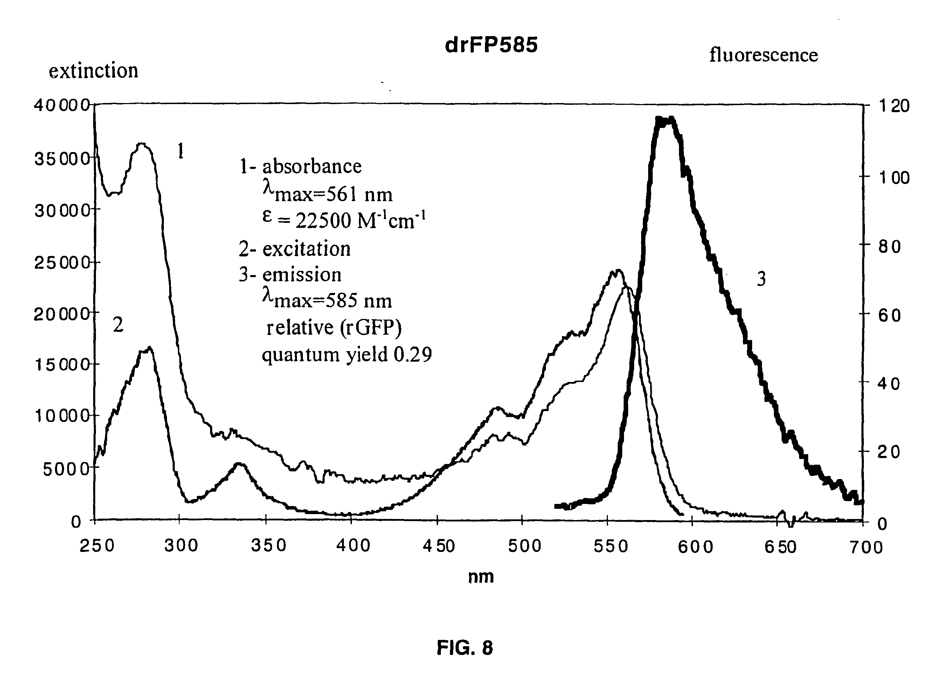

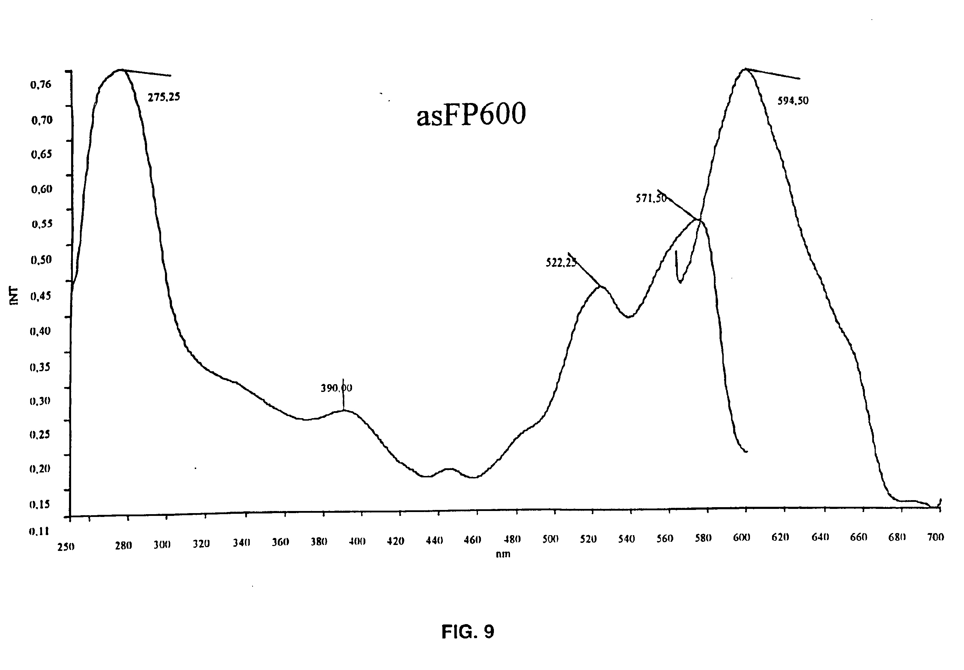

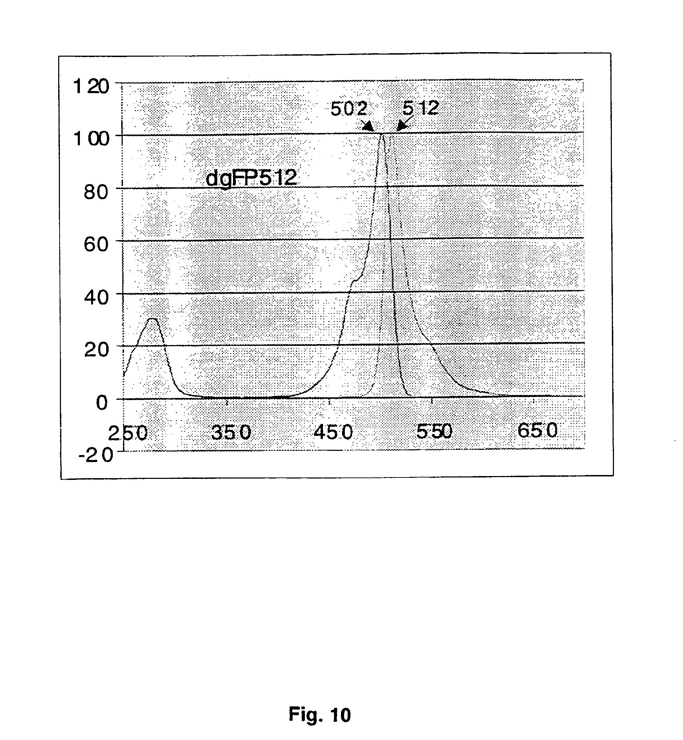

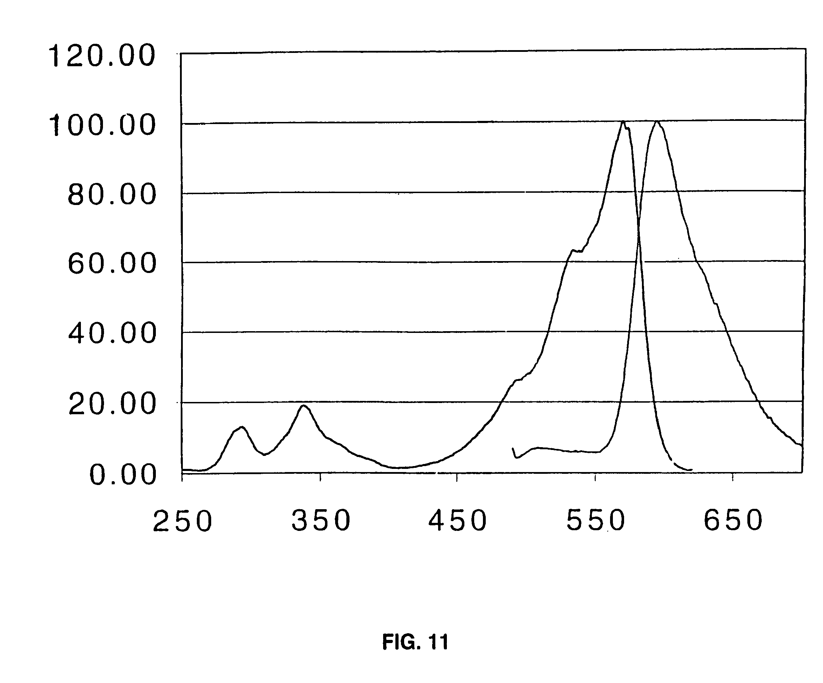

Nine cDNA full-length cDNAs encoding fluorescent proteins were obtained, and nine novel fluorescent proteins were produced (SEQ ID Nos. 55-63). The spectral properties of the isolated novel fluorescent proteins are shown in Table 7, and the emission and excitation spectra for the novel proteins are shown in Figures 3-11.

It is noted that the proteins as FP 600, dg FP512, and dm FP 592 (SEQ ID Nos. 61-63) are no embodiments of the present invention.

**TABLE 7**

| Spectral Properties of the Isolated NFPs. | | | | | | |
|---|---|---|---|---|---|---|
| Species | NFP Name | Abs. Max. n m | Emission Maximum n m | Maximum Extinction Coeff. | Relative Quantum Yield* | Relative Brightness ** |
| Anemonia majano | amFP486 | 458 | 486 | 40,000 | 0.3 | 0.43 |
| Clavularia sp. | cFP484 | 456 | 484 | 35,300 | 0.6 | 0.77 |
| Zoanthus sp. | zFP506 | 496 | 506 | 35,600 | 0.79 | 1.02 |
| Zoanthus sp. | zFP538 | 528 | 538 | 20,200 | 0.52 | 0.38 |
| Discosoma sp. "red" | drFP583 | 558 | 583 | 22,500 | 0.29 | 0.24 |
| Discosoma striata | dsFP483 | 443 | 483 | 23,900 | 0.57 | 0.50 |
| Anemonia sulcata | asFP600 | 572 | 596 | 56,200 | <0.001 | - |
| Discosoma sp "green" | dgFP512 | 502 | 512 | 20,360 | 0.3 | 0.21 |
| Discosoma sp. "magenta" | dmFP592 | 573 | 593 | 21,800 | 0.11 | 0.09 |

| | | | | | | |
|---|---|---|---|---|---|---|
| *relative quantum yield was determined as compared to the quantum yield of *A. victoria* GFP. **relative brightness is extinction coefficient multiplied by quantum yield divided by the same value for *A*. *victoria* G FP . | | | | | | |

Multiple alignment of fluorescent proteins is shown in Figure 2A. The numbering is based on *Aequorea victoria* green fluorescent protein (GFP, SEQ ID No. 54). The amino acid sequences of the novel fluorescent proteins are labeled as SEQ ID Nos. 55-63. Two proteins from *Zoanthus* and four from *Discosoma* are compared between each other: residues identical to the corresponding ones in the first proteins of the series are represented by dashes. Introduced gaps are represented by dots. In the sequence of *A. victoria* GFP, the stretches forming β-sheets are underlined; the residues whose side chains form the interior of the β-can are shaded. Figure 2B shows the N-terminal part of cFP484, which has no homology with the other proteins. The putative signal peptide is underlined.

The following references were cited herein.
1. Ormo et al., (1996) Science 273: 1392-1395.
2. Yang, F., et al., (1996) Nature Biotech 14: 1246-1251.
3. Cormack, et al., (1996) Gene 173, 33-38.
4. Haas, et al., (1996) Current Biology 6, 315-324.
5. Yang, et al., (1996) Nucleic Acids Research 24, 4592-4593.
6. Ghoda, et al.. (1990) J. Biol. Chem. 265: 11823-11826.
7. Prasher D.C. et al. (1992) Gene 111:229-33.
8. Kain et al. (1995) Biotechniques 19(4):650-55.
9. Chomczynski P., et al., (1987) Anal. Biochem. 162, 156-159.
10. Frohman et al., (1998) PNAS USA, 85, 8998-9002.

## Claims

1. A nucleic acid in other than its natural environment, the sequence of which encodes a fluorescent protein from a non-bioluminescent Anthozoan organism selected from the genera Anemonia, Clavularia, Zoanthus and Discosoma wherein:
(a) said fluorescent protein is from Anemonia majano and said nucleic acid can be amplified with a pair of primers, the first of which consists of the sequence of SEQ ID NO:36 and the second of which consists of the sequence of SEQ ID NO:37; or
(b) said fluorescent protein is from Clavularia sp. and said nucleic acid can be amplified with a primer pair consisting of a first primer which consists of the sequence of SEQ ID NO:38 and a second primer which consists of the sequence of SEQ ID NO:40: or
(c) said fluorescent protein is from Zoanthus sp. and said nucleic acid can be amplified with a primer pair consisting of a first primer which consists of the sequence of SEQ ID NO:41 and a second primer which consists of the sequence of SEQ ID NO:42; or
(d) said fluorescent protein is from Discosoma sp. "red" and said nucleic acid can be amplified with a primer pair consisting of a first primer which consists of the sequence of SEQ ID NO:43 and a second primer which consists of the sequence of SEQ ID NO:44; or
(e) said fluorescent protein is from Discosoma striata and said nucleic acid can be amplified with a primer pair consisting of a first primer which consists of the sequence of SEQ ID NO:45 and a second primer which consists of the sequence of SEQ ID NO:46.

2. A nucleic acid according to claim 1, wherein said fluorescent protein is from Anemonia majano and said nucleic acid can be amplified with a primer pair consisting of a first primer which consists of the sequence of SEQ ID NO:36 and a second primer which consists of the sequence of SEQ ID NO:37.

3. A nucleic acid according to claim 1, wherein said fluorescent protein is from Clavularia sp. and said nucleic acid can be amplified with a primer pair consisting of a first primer which consists of the sequence of SEQ ID NO:38 and a second primer which consists of the sequence of SEQ ID NO:40.

4. A nucleic acid according to claim 1, wherein said fluorescent protein is from Zoanthus sp. and said nucleic acid can be amplified with a primer pair consisting of a first primer which consists of the sequence of SEQ ID NO:41 and a second primer which consists of the sequence of SEQ ID NO:42.

5. A nucleic acid according to claim 1, wherein said fluorescent protein is from Discosoma sp. "red" and said nucleic acid can be amplified with a primer pair consisting of a first primer which consists of the sequence of SEQ ID NO:43 and a second primer which consists of the sequence of SEQ ID NO:44.

6. A nucleic acid according to claim 1, wherein said fluorescent protein is from Discosoma striata and said nucleic acid can be amplified with a primer pair consisting of a first primer which consists of the sequence of SEQ ID NO:45 and a second primer which consists of the sequence of SEQ ID NO:46.

7. The nucleic acid according to any one of claims 1 to 6 wherein said nucleic acid is isolated.

8. A construct comprising a vector and a nucleic acid according to any one of claims 1 to 6.

9. A cell transformed with a nucleic acid according to any one of claims 1 to 6.

10. A nucleic acid according to any one of claims 1 to 6 wherein the nucleic acid sequence can be amplified with a primer selected from the group consisting of SEQ ID NO: 4, 6, 7, 9, 10, 13, 15 and 16.

11. A fluorescent protein encoded by the nucleic acid according to any one of claims 1 to 6 present in other than its natural environment.

## Patentansprüche

1. Nucleinsäure in einer anderen als ihrer natürlichen Umgebung, deren Sequenz für ein fluoreszierendes Protein aus einem aus den Arten Anemonia, Clavularia, Zoanthus und Discosoma ausgewählten, nicht biolumineszierenden Anthozoenorganismus kodiert, worin:
(a) das fluoreszierende Protein von Anemonia majano stammt und die Nucleinsäure mit einem Paar Primern amplifiziert werden kann, von denen der erste aus der Sequenz von Seq.-ID Nr. 36 besteht und der zweite aus der Sequenz von Seq.-ID Nr. 37 besteht; oder
(b) das fluoreszierende Protein von Clavularia-Sp. stammt und die Nucleinsäure mit einem Primerpaar amplifiziert werden kann, das aus einem ersten Primer, der aus der Sequenz von Seq.-ID Nr. 38 besteht, und einem zweiten Primer besteht, der aus der Sequenz von Seq.-ID Nr. 40 besteht; oder
(c) das fluoreszierende Protein von Zoanthus-Sp. stammt und die Nucleinsäure mit einem Primerpaar amplifiziert werden kann, das aus einem ersten Primer, der aus der Sequenz von Seq.-ID Nr. 41 besteht, und einem zweiten Primer besteht, der aus der Sequenz von Seq.-ID Nr. 42 besteht; oder
(d) das fluoreszierende Protein von Discosoma-Sp. "rot" stammt und die Nucleinsäure mit einem Primerpaar amplifiziert werden kann, das aus einem ersten Primer, der aus der Sequenz von Seq.-ID Nr. 43 besteht, und einem zweiten Primer besteht, der aus der Sequenz von Seq.-ID Nr. 44 besteht; oder
(e) das fluoreszierende Protein von Discosoma striata stammt und die Nucleinsäure mit einem Primerpaar amplifiziert werden kann, das aus einem ersten Primer, der aus der Sequenz von Seq.-ID Nr. 45 besteht, und einem zweiten Primer besteht, der aus der Sequenz von Seq.-ID Nr. 46 besteht.

2. Nucleinsäure nach Anspruch 1, worin das fluoreszierende Protein von Anemonia majano stammt und die Nucleinsäure mit einem Primerpaar amplifiziert werden kann, das aus einem ersten Primer, der aus der Sequenz von Seq.-ID Nr. 36 besteht, und einem zweiten Primer besteht, der aus der Sequenz von Seq.-ID Nr. 37 besteht.

3. Nucleinsäure nach Anspruch 1, worin das fluoreszierende Protein von Clavularia-Sp. stammt und die Nucleinsäure mit einem Primerpaar amplifiziert werden kann, das aus einem ersten Primer, der aus der Sequenz von Seq.-ID Nr. 38 besteht, und einem zweiten Primer besteht, der aus der Sequenz von Seq.-ID Nr. 40 besteht.

4. Nucleinsäure nach Anspruch 1, worin das fluoreszierende Protein von Zoanthus-Sp. stammt und die Nucleinsäure mit einem Primerpaar amplifiziert werden kann, das aus einem ersten Primer, der aus der Sequenz von Seq.-ID Nr. 41 besteht, und einem zweiten Primer besteht, der aus der Sequenz von Seq.-ID Nr. 42 besteht.

5. Nucleinsäure nach Anspruch 1, worin das fluoreszierende Protein von Discosoma-Sp. "rot" stammt und die Nucleinsäure mit einem Primerpaar amplifiziert werden kann, das aus einem ersten Primer, der aus der Sequenz von Seq.-ID Nr. 43 besteht, und einem zweiten Primer besteht, der aus der Sequenz von Seq.-ID Nr. 44 besteht.

6. Nucleinsäure nach Anspruch 1, worin das fluoreszierende Protein von Discosoma striata stammt und die Nucleinsäure mit einem Primerpaar amplifiziert werden kann, das aus einem ersten Primer, der aus der Sequenz von Seq.-ID Nr. 45 besteht, und einem zweiten Primer besteht, der aus der Sequenz von Seq.-ID Nr. 46 besteht.

7. Nucleinsäure nach einem der Ansprüche 1 bis 6, worin die Nucleinsäure isoliert ist.

8. Konstrukt, umfassend einen Vektor und eine Nucleinsäure nach einem der Ansprüche 1 bis 6.

9. Zelle, transformiert mit einer Nucleinsäure nach einem der Ansprüche 1 bis 6.

10. Nucleinsäure nach einem der Ansprüche 1 bis 6, worin die Nucleinsäuresequenz mit einem aus der aus den Seq.-ID Nr. 4, 6, 7, 9, 10, 13, 15 und 16 bestehenden Gruppe ausgewählten Primer amplifiziert werden kann.

11. Fluoreszierendes Protein, für das eine Nucleinsäure nach einem der Ansprüche 1 bis 6, die sich in einer anderen als ihrer natürlichen Umgebung befindet, kodiert.

## Revendications

1. Acide nucléique dans un autre que son environnement naturel, dont la séquence code pour une protéine fluorescente d'un organisme d'Anthozoane non-bioluminescent sélectionné parmi les espèces Anemonia, Clavularia, Zoanthus et Discosoma, où:
(a) ladite protéine fluorescente est d'une Anemonia majano, et ledit acide nucléique peut être amplifié avec une paire d'amorces, dont la première consiste en la séquence de SEQ ID NO:36, et dont la seconde consiste en la séquence de SEQ ID NO: 37; ou
(b) ladite protéine fluorescente est de Clavularia sp, et ledit acide nucléique peut être amplifié avec une paire d'amorces consistant en une première amorce qui consiste en la séquence de SEQ ID NO: 38 et une seconde amorce qui consiste en la séquence de SEQ ID NO: 40; ou
(c) ladite protéine fluorescente est de Zoanthus sp. et ledit acide nucléique peut être amplifié avec une paire d'amorces consistant en une première amorce qui consiste en la séquence de SEQ ID NO: 41 et une seconde amorce qui consiste en la séquence de SEQ ID NO: 42; ou
(d) ladite protéine fluorescente est de Discosoma sp. "rouge", et ledit acide nucléique peut être amplifié avec une paire d'amorces consistant en une première amorce qui consiste en la séquence de SEQ ID NO: 43 et une seconde amorce qui consiste en la séquence de SEQ ID NO: 44; ou
(e) ladite protéine fluorescente est de Discosoma striata, et ledit acide nucléique peut être amplifié avec une paire d'amorces consistant en une première amorce qui consiste en la séquence de SEQ ID NO: 45 et une seconde amorce qui consiste en la séquence de SEQ ID NO: 46.

2. Acide nucléique selon la revendication 1, dans lequel ladite protéine fluorescente est de Anemonia majano, et ledit acide nucléique peut être amplifié avec une paire d'amorces consistant en une première amorce qui consiste en la séquence de SEQ ID NO:36 et une seconde amorce qui consiste en la séquence de SEQ ID NO:37.

3. Acide nucléique selon la revendication 1, dans lequel ladite protéine fluorescente est de Clavularia sp., et ledit acide nucléique peut être amplifié avec une paire d'amorces consistant en une première amorce qui consiste en la séquence de SEQ ID NO:38 et une seconde amorce qui consiste en la séquence de SEQ ID NO:40.

4. Acide nucléique selon la revendication 1, dans lequel ladite protéine fluorescente est de Zoanthus sp., et ledit acide nucléique peut être amplifié avec une paire d'amorces consistant en une première amorce qui consiste en la séquence de SEQ ID NO:41 et une seconde amorce qui consiste en la séquence de SEQ ID NO:42.

5. Acide nucléique selon la revendication 1, dans lequel ladite protéine fluorescente est de Discosoma sp. "rouge", et ledit acide nucléique peut être amplifié avec une paire d'amorces consistant en une première amorce qui consiste en la séquence de SEQ ID NO:43 et une seconde amorce qui consiste en la séquence de SEQ ID NO:44.

6. Acide nucléique selon la revendication 1, dans lequel ladite protéine fluorescente est de Discosoma striata, et ledit acide nucléique peut être amplifié avec une paire d'amorces consistant en une première amorce qui consiste en la séquence de SEQ ID NO:45 et une seconde amorce qui consiste en la séquence de SEQ ID NO:46.

7. Acide nucléique selon l'une quelconque des revendications 1 à 6, où ledit acide nucléique est isolé.

8. Produit de synthèse comprenant un vecteur et un acide nucléique selon l'une quelconque des revendications 1 à 6.

9. Cellule transformée par un acide nucléique selon l'une quelconque des revendications 1 à 6.

10. Acide nucléique selon l'une quelconque des revendications 1 à 6, où la séquence d'acides nucléiques peut être amplifiée avec une amorce sélectionnée dans le groupe consistant en SEQ ID NO:4, 6, 7, 9, 10, 13, 15 et 16.

11. Protéine fluorescente codée par l'acide nucléique selon l'une quelconque des revendications 1 à 6 présente dans un autre que son environnement naturel.
